# EUROPEAN PATENT APPLICATION

(11) **EP 1 533 383 A1**
(43) Date of publication of application: **25.05.2005**
(21) Application number: 03741246.7
(22) Date of filing: 07.07.2003
(51) Int. Cl.: C12Q 1/02, C12N 15/09, A61K 45/00, G01N 33/53, G01N 33/566

(54) **NOVEL METHOD OF SELECTING IMMUNOSUPPRESSANT HAVING LITTLE THROMBOCYTOPENIC EFFECT**

(30) Priority: 12.07.2002 JP 2002203901
(71) Applicant: FUJISAWA PHARMACEUTICAL CO., LTD., Osaka-shi, Osaka-fu 541-8514 (JP)
(72) Inventor: FUJIMURA, T.; c/o Fujisawa Pharmaceutical Co. Ltd, Osaka-shi, Osaka 541-8514 (JP); MORI, H.; c/o Fujisawa Pharmaceutical Co. Ltd, Osaka-shi, Osaka 541-8514 (JP); YOSHIZAWA, K.; c/o Fujisawa Pharmaceutical Co. Ltd, Osaka-shi, Osaka 541-8514 (JP); TAKADA, Y.; c/o Fujisawa Pharmaceutical Co. Ltd, Osaka-shi, Osaka 541-8514 (JP); ARAMORI, I., c/o Fujisawa Pharmaceutical Co., Ltd, Osaka-shi, Osaka 541-8514 (JP); MATSUOKA, H., c/o Fujisawa Pharmaceutical Co.,Ltd, Osaka-shi, Osaka 541-8514 (JP); UNAMI, Akira, c/o Fujisawa Pharmaceutical Co., Ltd, Osaka-shi, Osaka 541-8514 (JP); NOTO, T., c/o Fujisawa Pharmaceutical Co., Ltd, Osaka-shi, Osaka 541-8514 (JP)
(74) Representative: Gille Hrabal Struck Neidlein Prop Roos
(86) International application number: PCT/JP2003/008621
(87) International publication number: WO 2004/007758

(57) **Abstract**

The invention relates to a novel method for selecting an immunosuppressive agent with a less thrombocytopenia effect. According to the invention, a method for selecting an immunosuppressive agent which has a potent immunosuppressive activity but a lower thrombocytopenia effect, said method comprising measuring an IL-2 transcription inhibitory activity in a test cell into which an IL-2 reporter gene has been introduced in the coexistence of an analyte, while measuring a GATA-1 transcription inhibitory activity in the test cell into which a GATA-1 reporter gene has been introduced in the coexistence of an analyte, and comparing both the transcription inhibitory activities, is provided.

SEQUENCE LISTING

<110> Fujisawa Pharmaceutical Co., Ltd.

<120> New methods for selecting an immunosuppressive agent having the reduced
risk of causing thrombocytopenia as its side effect

<130> PWO23998

<140> PCT/JP03/08621
<141> 2003-07-07

<150> JP 2002-203901
<151> 2002-07-12

<160> 17

<170> PatentIn version 3.1

<210> 1
<211> 32
<212> DNA
<213> Artificial Sequence

<220>
<223> Description of Artificial Sequence: PCR primer-1

<400> 1
tcgctagcct gagtatttaa caatcgcacc ct 32


<210> 2
<211> 30
<212> DNA
<213> Artificial Sequence

<220>
<223> Description of Artificial Sequence: PCR primer- 2

<400> 2
cgaagcttgt ggcaggagtt gaggttactg 30


<210> 3
<211> 30
<212> DNA
<213> Artificial Sequence

<220>
<223> Description of Artificial Sequence: PCR primer-3

<400> 3
cgctagctgc tcttgtccac cacaatatgc 30


<210> 4
<211> 28
<212> DNA
<213> Artificial Sequence

<220>
<223> Description of Artificial Sequence: PCR primer-4

<400>  4
atagatctat ccctggctcc cacctcag                                          28


<210>  5
<211>  28
<212>  DNA
<213>  Artificial Sequence

<220>
<223>  Description of Artificial Sequence:  PCR primer-5

<400>  5
ataagctttg gtggttgcgg agggttcg                                          28


<210>  6
<211>  28
<212>  DNA
<213>  Artificial Sequence

<220>
<223>  Description of Artificial Sequence: PCR primer-6

<400>  6
atggtaccac cccagaagat gccaggag                                          28


<210>  7
<211>  28
<212>  DNA
<213>  Artificial Sequence

<220>
<223>  Description of Artificial Sequence: PCR primer- 7

<400>  7
atgctagcgc cctctgagcc tcagtttc                                          28


<210>  8
<211>  731
<212>  DNA
<213>  Homo sapiens

<220>
<221>  misc_feature
<222>  (1)..(731)
<223>  Human interleukin-2 (IL-2)  gene  5'-flanking region


<220>
<221>  TATA_signal
<222>  (652)..(657)
<223>

<220>
<221>  misc_feature
<222>  (676)..(657)
<223>  The putative transcription initiation site of the IL-2 gene promo
       ter

<220>
<221>  misc_feature
<222>  (1)..(731)
<223>  Corresponding to the sequence (689-1416) in  the GenBank database
       (Acession: X00695), except for several variations.

<220>
<221>  variation
<222>  (38)..(731)
<223>  1 bp insertion

<220>
<221>  variation
<222>  (196)..(731)
<223>  T to A exchange

<220>
<221>  variation
<222>  (346)..(731)
<223>  T to G exchange

<220>
<221>  variation
<222>  (577)..(731)
<223>  T to A exchange

<220>
<221>  variation
<222>  (688)..(689)
<223>  2 bp insertion  (CT)

<400>  8
ctgagtattt aacaatcgca ccctttaaaa aatgtacaat agacattaag agacttaaac      60

agatatataa tcattttaaa ttaaaatagc gttaaacagt acctcaagct caataagcat     120

tttaagtatt ctaatcttag tatttctcta gctgacatgt aagaagcaat ctatcttatt     180

gtatgcaatt agctcattgt gtggataaaa aggtaaaacc attctgaaac aggaaaccaa     240

tacacttcct gtttaatcaa caaatctaaa catttattct tttcatctgt ttactcttgc     300

tcttgtccac cacaatatgc tattcacatg ttcagtgtag ttttaggaca aagaaaattt     360

tctgagttac ttttgtatcc ccaccccctt aaagaaagga ggaaaaactg tttcatacag     420

aaggcgttaa ttgcatgaat tagagctatc acctaagtgt gggctaatgt aacaaagagg     480

gatttcacct acatccattc agtcagtctt tgggggttta aagaaattcc aaagagtcat     540

cagaagagga aaaatgaagg taatgttttt tcagacaggt aaagtctttg aaaatatgtg     600

taatatgtaa aacattttga cacccccata atatttttcc agaattaaca gtataaattg     660

catctcttgt tcaagagttc cctatcactc tctttaatca ctactcacag taacctcaac     720

tcctgccaca a                                                          731

<210>  9
<211>  819
<212>  DNA
<213>  Homo sapiens

<220>
<221>  misc_feature
<222>  (1)..(819)
<223>  Human GATA- 1 gene promoter region

<220>
<221>  misc_feature
<222>  (1)..(819)
<223>  Corresponding to the sequence (5342-6160) reported by  K. Blechsc
       hmidt et al. /  GenBank (Accession: AF196971)

<220>
<221>  misc_feature
<222>  (790)..(819)
<223>  The putative transcription initiation site of the GATA-1 gene pro
       moter

<400>  9
atccctggct cccacctcag tttcccgcct ccaaggcagc atggcgggca agaagttgag      60

gccactgtcc ctgggtgttc ctacccccac accctcaccc caagacagcc tgttactgcg     120

gcgccaacag ccacggtcgc ctacatctga taagacttat ctgctgcccc agggcaggcc     180

ggagctggcg taagccccag tggggcgcta agtgagtgtg cccctgcctc ccgccagcac     240

tggcctggcc tgcaggctta gcctgggtca tcaaggtatc ccacaggctc tagttcaaat     300

ccagcagaac ctctctgagc ctcactcttc tcacctgcaa aatgggtaca gccacatccc     360

ttctctccct gcagccagga agacgcacat acacaggagt ctagcccaca ccggccccgc     420

acaaattaag ggctttactc tctgaaaagc ccagtgaagt catgaaacca tatctgctat     480

tttcatttat cttggtttca gcctattttg cttgtctgga cactacagtc cacgggagcc     540

taggtcgagc gaggtccaag aatccccagg gtgggcaggg agggtggaag agggcctcca     600

gtgcccaaga ggtgccccac aagcatggga cccgccccct cccctggact gccccaccca     660

ctggggcacc agccactccc tggggaggag ggaggaggga gaagggaggg agggagggag     720

ggaggaaggg agcctcaaag gccaaggcca gccaggacac cccctgggat cacactgagc     780

ttgccacatc cccaaggcgg ccgaaccctc cgcaaccac                           819

<210>  10
<211>  637
<212>  DNA

<213>  Homo sapiens

<220>
<221>  misc_feature
<222>  (1)..(637)
<223>  Human GATA- 1 gene enhancer region


<220>
<221>  misc_feature
<222>  (1)..(637)
<223>  Corresponding to the sequence (2362-2998)  reported by  K. Blechs
       chmidt et al.  /   GenBank (Accession: AF196971)


<400>  10
   accccagaag atgccaggag ggagtgagcc agtcagggaa ggcttccgag aagagaggac     60
   attgaagaag agtctcaaac ttaggcctga cggagaagac gcgcggccag gacaccccac    120
   ccccgccctc gtctccccca aagcctgatc tggccccact gattccctta tctgcccact    180
   cccagctgcc tccttgctgg ctgaactgtc gccgcagact tctgagcctg cgccccctcc    240
   acggggatgg gggagggaat ggggtgaggc ctggcctcac agcctcgggg tttccagctc    300
   ttgctggagg cagggctctg gggcgcccta ctcctcaccc ttggcttctc ttcctgagcg    360
   ctctgtgctc tccagaaatg aagaaatggg gtgagtccag cggccaaacc cttgtcttag    420
   ctcttagaca tgcctcgagc ctgccattcc ctgtgaggac agatttccct atgttgcgac    480
   cgctgcttct aataataata atgatgatga taattcccat ttacagagca caccatttat    540
   ggtgtgccag caggccctgt gctgagtggt tcctacccac gtggggggct aggactttac    600
   ccgttttcca gatgaagaaa ctgaggctca gagggcg                             637


<210>  11
<211>  434
<212>  DNA
<213>  Homo sapiens

<220>
<221>  misc_feature
<222>  (1)..(434)
<223>  Human interleukin-2 (IL-2) gene  5'-flanking region


<220>
<221>  TATA_signal
<222>  (355)..(360)
<223>

<220>
<221>  misc_feature
<222>  (379)..(360)
<223>  The putative transcription initiation site of the IL-2 gene promo
       ter

<220>
<221>  misc_feature
<222>  (1)..(434)
<223>  Corresponding to the sequence (+985 to +1416 ) in the GenBank dat
       abase (Acession: X00695), except for several variations.

<220>
<221>  variation
<222>  (49)..(434)
<223>  T to G exchange

<220>
<221>  variation
<222>  (280)..(434)
<223>  T to A exchange

<220>
<221>  variation
<222>  (391)..(392)
<223>  2 bp insertion (CT)

<400>  11
tgctcttgtc caccacaata tgctattcac atgttcagtg tagttttagg acaaagaaaa      60

ttttctgagt tacttttgta tccccacccc cttaaagaaa ggaggaaaaa ctgtttcata     120

cagaaggcgt taattgcatg aattagagct atcacctaag tgtgggctaa tgtaacaaag     180

agggatttca cctacatcca ttcagtcagt ctttgggggt ttaaagaaat tccaaagagt     240

catcagaaga ggaaaaatga aggtaatgtt ttttcagaca ggtaaagtct ttgaaaatat     300

gtgtaatatg taaaacattt tgacaccccc ataatatttt tccagaatta acagtataaa     360

ttgcatctct tgttcaagag ttccctatca ctctctttaa tcactactca cagtaacctc     420

aactcctgcc acaa                                                       434

<210>  12
<211>  59
<212>  DNA
<213>  Artificial Sequence

<220>
<223>  CEBP-11, synthetic DNA

<400>  12
cgcgttgagc aagacttgag caagtacttg agcaagcgtt gagcaaggct tgagcaagc      59

<210>  13
<211>  59
<212>  DNA
<213>  Artificial Sequence

<220>

<223>  CEBP-12, synthetic DNA

<400>  13
tcgagcttgc tcaagccttg ctcaacgctt gctcaagtac ttgctcaagt cttgctcaa      59


<210>  14
<211>  51
<212>  DNA
<213>  Artificial Sequence

<220>
<223>  HSE-11, synthetic DNA

<400>  14
cgcgtctaga atgttctaga tctagaacat tctagctaga atgttctaga c              51


<210>  15
<211>  51
<212>  DNA
<213>  Artificial Sequence

<220>
<223>  HSE-12, synthetic DNA

<400>  15
tcgagtctag aacattctag ctagaatgtt ctagatctag aacattctag a              51


<210>  16
<211>  651
<212>  DNA
<213>  Artificial Sequence

<220>
<223>  GATA-1 gene HSI region (mutant)

<220>
<221>  mutation
<222>  (178)..(178)
<223>  in vitro mutation (from "a" to  "g")


<220>
<221>  mutation
<222>  (519)..(519)
<223>  in vitro mutation (from "t" to  "g")


<400>  16
ctacgcgtac cccagaagat gccaggaggg agtgagccag tcagggaagg cttccgagaa      60

gagaggacat tgaagaagag tctcaaactt aggcctgacg gagaagacgc gcggccagga     120

caccccaccc ccgccctcgt ctcccccaaa gcctgatctg gccccactga ttcccttgtc     180

tgcccactcc cagctgcctc cttgctggct gaactgtcgc cgcagacttc tgagcctgcg     240

ccccctccac ggggatgggg gagggaatgg ggtgaggcct ggcctcacag cctcggggtt     300

tccagctctt gctggaggca gggctctggg gcgccctact cctcaccctt ggcttctctt    360

cctgagcgct ctgtgctctc cagaaatgaa gaaatggggt gagtccagcg gccaaaccct    420

tgtcttagct cttagacatg cctcgagcct gccattccct gtgaggacag atttccctat    480

gttgcgaccg ctgcttctaa taataataat gatgatgaga attcccattt acagagcaca    540

ccatttatgg tgtgccagca ggccctgtgc tgagtggttc ctacccacgt ggggggctag    600

gactttaccc gttttccaga tgaagaaact gaggctcaga gggcagatct g    651

<210>  17
<211>  838
<212>  DNA
<213>  Artificial Sequence

<220>
<223>  GATA-1 gene IE promoter (mutant)

<220>
<221>  mutation
<222>  (160)..(160)
<223>  in vitro mutation (from "t" to "g")

<220>
<221>  mutation
<222>  (168)..(168)
<223>  in vitro mutation (from "a" to "g")

<220>
<221>  mutation
<222>  (287)..(287)
<223>  in vitro mutation (from "a" to "g")

<220>
<221>  mutation
<222>  (482)..(482)
<223>  in vitro mutation (from "t" to "g")

<220>
<221>  mutation
<222>  (498)..(498)
<223>  in vitro mutation (from "a" to "g")

<220>
<221>  mutation
<222>  (798)..(798)
<223>  in vitro mutation (from "t" to "g")

<400>  17
atagatctga tccctggctc ccacctcagt ttcccgcctc caaggcagca tggcgggcaa    60

gaagttgagg ccactgtccc tgggtgttcc tacccccaca ccctcacccc aagacagcct    120

gttactgcgg cgccaacagc cacggtcgcc tacatctgag aagacttgtc tgctgcccca 180

gggcaggccg gagctggcgt aagccccagt ggggcgctaa gtgagtgtgc ccctgcctcc 240

cgccagcact ggcctggcct gcaggcttag cctgggtcat caaggtgtcc cacaggctct 300

agttcaaatc cagcagaacc tctctgagcc tcactcttct cacctgcaaa atgggtacag 360

ccacatccct tctctccctg cagccaggaa gacgcacata cacaggagtc tagcccacac 420

cggccccgca caaattaagg gctttactct ctgaaaagcc cagtgaagtc atgaaaccat 480

agctgctatt ttcatttgtc ttggtttcag cctattttgc ttgtctggac actacagtcc 540

acgggagcct aggtcgagcg aggtccaaga atccccaggg tgggcaggga gggtggaaga 600

gggcctccag tgcccaagag gtgccccaca agcatgggac ccgccccctc ccctggactg 660

ccccacccac tggggcacca gccactccct ggggaggagg gaggagggag aagggaggga 720

gggagggagg gaggaaggga gcctcaaagg ccaaggccag ccaggacacc ccctgggatc 780

acactgagct tgccacagcc ccaaggcggc cgaaccctcc gcaaccacca aagcttat 838

## Description

### Technical Field

The present invention relates to a novel method for selecting an immunosuppressive agent having the reduced risk of causing thrombocytopenia.

### Background Art

Major immunosuppressive agents, cyclosporin A (CsA) and tacrolimus (KF506), which have now widely been used in clinical fields in order to suppress acute rejection after organ transplantation, inhibit the activity of calcineurin, a Ca²⁺/calmodulin-dependent protein phosphatase, through binding to respectively specific immunophilins (for example, cyclophilin for CsA and FKBP12 for FK506). Consequently, it is known that the intranuclear transfer of NF-AT (nuclear factor of activated T-cell) is inhibited by inhibition of dephosphorylation of NF-AT, resulting in suppression of the transcriptional activity of IL-2 gene. From the study of such action mechanism, it has become apparent that the expression of IL-2 gene at the transcriptional level is inhibited in the activated T-cells to suppress the rejection of the graft in organ transplantation, and this is very important in obtaining a therapeutic effect in various autoimmune diseases.

In this connection, it has been known that a series of histone deacetylases (hereinafter referred to as HDAC) that catalyze histone deacetylation work competitively with histone acetylases in a cell nucleus to control the expression level of various genes through alteration of the chromatin structure. As the results of so far energetic screening, a large number of HDAC-inhibitory compounds have been provided, which include compounds remarkably inhibiting the production of IL-2 (I. Takahashi et al., (1995) The Journal of Antibiotics 49, 453-457) and have attracted a considerable attention as candidates of immunosuppressive agents complementing cyclosporin A and tacrolimus. In fact, among thus chosen compounds, some ones showing an excellent in vivo immunosuppressive effect have been found. For example, as disclosed in WO 00/08048, FR225497 has been found to show an excellent effect as a therapeutic or preventive agent for organ transplant rejection or autoimmune diseases through the potent immunosuppressive effect, and in addition, it is suggested to have usefulness as a therapeutic or preventive agent for many other diseases which are considered to onset due to abnormal expression of genes. Such diseases include, for example, inflammatory disorders, diabetes mellitus, diabetic complication, homozygous thalassemia, fibrosis, cirrhosis, acute promyelocytic leukemia (APL), protozoal infection, tumor, and the like. Even though such usefulness has been suggested, however, there is a problem that many of these HDAC-inhibitory compounds would sometimes have such an adverse reaction as serious thrombocytopenia when administered to the living body, making it difficult to use as a practical therapeutic agent.

The cause that many of immunosuppressive HDAC inhibitors readily produce such a side effect as thrombocytopenia has not yet been elucidated sufficiently. The present inventors, however, have found in their assiduous study that many of HDAC inhibitory compounds also inhibit the transcriptional activity of GATA-1 (also called GATA-1 binding protein, GF-1, NF-E1 or Eryf 1) gene.

GATA-1 is a DNA binding protein which recognizes a (A/T) GATA (A/G) consensus sequence characteristically existing in the transcriptional region of hemopoietic gene. This GATA motif sequence has been found in a variety of regulatory regions or promoters such as enhancer regions of various globin genes, locus control regions (LCRs) of β-globin genes, T-cell receptor α-chain, or enhancer regions of δ-chain genes. In addition, GATA-1 mRNA has been highly expressed in mature erythrocytes, mast cells or megakaryocytes and slightly expressed in polyfunctional precursor cells, or the testicle of young mouse.

In Gata-1 protein, there are 2 sites of C4-type Zn (zinc) finger region. Among them, the Zn finger existing on the N-terminal end has been known to carry the essential function for mature of erythrocytes and megakaryocytes through inter act ion with a transcriptional coupling factor such as Fog-1 (friend of GATA-1). For example, it has been reported that, though the human GATA-1 gene is resident on X chromosome, a mutation of V205M or D218G can be recognized at the site corresponding to the Zn finger in some patients suffering from X-chromosomal associated hereditary dyshemopoietic anemia or thrombocytopenia (K.E. Nichols et al., (2000) Nat. Genet. 24, 266-270; K. Freson et al., (2001) Blood 98, 85-92). Fog-1 per se has 9 Zn fingers, probably through which it is estimated to play a role in mediating the binding between the GATA-1 linking to a promoter region and the other nuclear factor.

Regarding the promoter of GATA-1 gene itself, at least two promoters, i.e., IE promoter and IT promoter, have so far been found. Correspondingly, there are 2 first exons in this gene. In these exons, it is said that IT promoter specifically acts in the testicle Sertoli cells and IE promoter act mainly in hematopoietic cells. However, it has been reported that IT promoter also acts at the step of differentiation of primary erythroid cells (A.M. Vannucchi et al., (1999) Journal of Cellular Physiology 180, 390-401), but it has not yet been elucidated fully how the 2 promoters are chosen *in vivo* for action.

In the transcriptional control in hematopoietic cells, the IE promoter sequence which is estimated to exist in the range up to around 0.7 kb upstream of the transcription initiation point is considered important. In this region, though there is a sequence coincident with GATA motif or CACC motif, the details of the control sequence present therein are almost unknown since direct analysis on the human sequence has been reported very little. However, the transcriptional activity in this region alone is very weak, and accordingly it is considered to need the HSI region of about 317 base pairs (highly sensitive region of DNase I) existing at around 3.8kb to 3.5kb further upstream, at least in expression in megakaryocytes (P. Vyas et al., (1999) Development 126, 2799-2811; S. Nishimura et al., (2000) Molecular and Cellular Biology 20, 713-723). In the HSI region, there is a GATA-E-box motif in which the GATA motif sequence is proximate to the E-box motif sequence; this suggests that the GATA factor such as GATA-1, GATA-2, etc., possibly forms a complex with a nuclear protein, for example, SCL/tal-1, E2A (TCF3, transcription factor 3), LMO-2 (LIM only protein 2) or Ldb-1 (LIM domain binding factor-1) for interaction. In the sequence of the HSI region, there is a well conserved region between human and mouse.

In a mouse whose GATA-1 gene has been knocked out in a usual way, malformation of primary hematopoietic cells causes lethal damage at the stage of blastogenesis (Y. Fujiwara et al., (1996) Proc. Natl. Acad. Sci. USA 93, 12355-12358). On the other hand, it is possible to prepare a knock-out mouse in which the expression of GATA-1 gene of megakaryocyte line is selectively knocked out; in such a mouse, it has been found that the number of platelet is sharply reduced and the normal maturation of megakaryocytic cells is not recognized (R.A. Shivdasani et al., (1997) The EMBO Journal 16, 3965-3973).

### Disclosure of Invention

To date, a large number of HDAC inhibitory compounds exhibiting an excellent immunosuppressive effect when administered to the living body, are known, but these compounds are not necessarily satisfactory because they have serious thrombocytopenia at the same time, making it difficult to use clinically. Thus, it has strongly been desired to provide a good method for in vitro screening a compound from these candidates which has no thrombocytopenia effect. The purpose of the invention is to solve such a problem.

From the studies to date, it has been found that the GATA-1 gene product plays an important role in differentiation and maturation of erythrocytes and megakaryocytic cells (X. Tang et al., (2001) : CMLS, Cell. Mol. Life Sci. 58, 2008-2017). In addition to GATA-1, however, many other factors have been suggested to be involved in differentiation and maturation of megakaryocytic cells; and it was really unclear whether the thrombocytopenia effect often recognized in many of HDAC inhibitors is through inhibition of the function of GATA-1 itself. According to the present inventor's study, it was first elucidated, as a result of comparative study on the effect of various HDAC inhibitors, that the thrombocytopenia effect caused by administration of these drugs is based on suppression of the transcription of GATA-1 gene. In order to search the cause of thrombocytopenia effect observed in many of HDAC inhibitors, the present inventors, using GeneChip^{(R)} (Affymetrix), screened and grouped a human-originated gene of which the transcription was inhibited by an HDAC inhibitor in the same pattern as GATA-1 from the genes expressing in megakaryocytic cells, and searched a transcriptional factor common to these gene groups. And, from 45 genes of which the transcription was inhibited by an HDAC inhibitor in the same pattern as GATA-1 gene, the inventors obtained 10 genes as candidates for a transcriptional factor expected to have the function involved in differentiation of blood corpuscles or as candidates expected to be megakaryocytic marker genes. Then, the inventors examined in details whether or not a binding sequence of a transcriptional factor commonly present in the transcriptional control region of the respective genes and that of the GATA-1 gene exists, and they found that there is a responsive sequence recognized by STAT3, C/EBPβ and HSF1 almost commonly in addition to the responsive sequence recognized by GATA-1 itself (GATA sequence). The inventors then constructed an expression system for a reporter gene which is regulated by the responsive sequence recognized by other respective transcriptional factors than the above-mentioned GATA-1, and examined the effect with HDAC inhibitors. In any cases, it was found that the transcription of reporter gene was not inhibited by HDAC inhibitors.

Further, the present inventors constructed artificially a promoter in which a responsive sequence recognized by GATA-1 itself was lost, by introducing a mutation into the GATA sequence present in the GATA-1 gene promoter. This was integrated in an expression system for a reporter gene and transformed into cells to examine. As a result, it was found that transcription of the reporter gene was not inhibited by at least 3 members of HDAC inhibitors. These results indicate that the inhibition of GATA-1 gene expression by HDAC inhibitors is caused by inhibition of the function of transcriptional activation by transcriptional factors through a GATA sequence in the GATA-1 gene promoter. That is, these results strongly suggest a possibility that the inhibition by an HDAC inhibitor of the transcriptional activation function induced by a GATA-1 factor might generate an adverse effect, thrombocytopenia, since the GATA-1 factor *per se* exhibits the transcriptional activation function through the GATA sequence.

In this connection, the present inventors have eagerly studied an effective dose of a large number of HDAC inhibitors showing an immunosuppressive activity on a rat's heart transplantation model, and they have realized that the efficacy as an immunosuppressive agent in the rat's heart transplantation model is deeply associated with inhibition of the IL-2 gene expression. In order to confirm this fact, a reporter assay system was constructed using a transcriptional control region of IL-2 gene. A DNA construct containing the reporter gene was introduced into an activated cell strain derived from T-cell, and a change of the reporter activity was measured and compared with addition of a variety of compounds to be tested. As a result, it was found that the efficacy as an immunosuppressive agent in the rat's heart transplantation model correlated well with the inhibition of IL-2 transcription.

Further, the present inventors have realized that the serious side effect of a large number of HDAC inhibitors, i.e., thrombocytopenia effect is deeply associated with the inhibition of expression of GATA-1 gene. In order to confirm this fact, a reporter assay system was constructed using a transcriptional control region of GATA-1 gene. A DNA construct containing these reporter genes was introduced into a cell strain derived from a megakaryocytic cells, and a change of the reporter activity was measured and compared with addition of a variety of compounds (analytes) to be tested. As a result, it was found that there was a tendency that a compound having a stronger platelet inhibitory activity strongly inhibited the transcription of GATA-1 gene.

In this connection, the present inventors have found a method for selecting an immunosuppressive agent with a less thrombocytopenia effect, the method comprising making an analyte coexist with a test cell into which a GATA-1 reporter gene has been introduced, and measuring the transcription inhibitory activity of the GATA-1 in the test cell.

Further, the inventors invented a method for rapidly selecting a compound with a less thrombocytopenia effect as a side effect and with an immunosuppressive activity, the method comprising selecting a compound having a strong inhibitory activity for IL-2 transcription and a weak inhibitory activity for GATA-1 transcription by simultaneously using a reporter assay system utilizing the above 2 species of cells.

The invention relates to a method for selecting an immunosuppressive agent with a less thrombocytopenia effect, the method comprising measuring an IL-2 transcription inhibitory activity in a test cell into which an IL-2 reporter gene has been introduced in the coexistence of an analyte, while measuring a GATA-1 transcription inhibitory activity in the test cell into which a GATA-1 reporter gene has been introduced in the coexistence of an analyte, and comparing both the transcription inhibitory activities.

Further, the invention relates to a method for selecting an immunosuppressive agent with a less thrombocytopenia effect, the method comprising measuring the amount of expressed IL-2 protein, measuring the amount of expressed GATA-1 protein, and comparing both the amounts of expression.

Further, the invention relates to a kit for selecting an immunosuppressive agent with a less thrombocytopenia effect, the kit comprising a DNA construct containing an IL-2 reporter gene, a DNA construct containing a GATA-1 reporter gene, a test cell of T-cell line, and a test cell of megakaryocytic cell line.

Further, the invention relates to a therapeutic agent for treatment of inflammatory disorders, diabetes mellitus, diabetic complications, homozygous thalassemia, fibrosis, cirrhosis, acute promyelocytic leukemia (APL), protozoal infections, organ transplant rejection, autoimmune diseases, and tumors, the agent comprising as an active ingredient a compound selected by measuring the (IL-2 IC50) value as an IL-2transcription inhibitory activity, measuring the (GATA-1 IC50) value as a GATA-1transcription inhibitory activity, comparing both the values, and selecting a compound having the (GATA-1 IC50)/(IL-2 IC50) value of 5 or more.

That is, the present invention relates to the following ones.
<1> A method for selecting an immunosuppressive agent with a less thrombocytopenia effect, the method comprising the following items (1) and (2):
   (1) selecting compounds having an immunosuppressive activity; and
   (2) selecting a compound having a weak GATA-1 transcription inhibitory activity from the compounds selected in (1).
<2> A method for selecting an immunosuppressive agent with a less thrombocytopenia effect, the method comprising the following items (1) to (3):
   (1) measuring an immunosuppressive activity of an analyte;
   (2) measuring a GATA-1 transcription inhibitory activity of the analyte; and
   (3) comparing the immunosuppressive activity determined in (1) with the GATA-1 transcription inhibitory activity determined in (2) to select an immunosuppressive agent with a less thrombocytopenia effect.
<3> A method for selecting an immunosuppressive agent with a less thrombocytopenia effect, the method comprising the following items (1) to (3):
   (1) measuring an IL-2 transcription inhibitory activity in a test cell in the coexistence of the test cell and an analyte;
   (2) measuring an GATA-1 transcription inhibitory activity in a test cell in the coexistence of the test cell and an analyte; and
   (3) comparing the IL-2 transcription inhibitory activity determined in (1) with the GATA-1 transcription inhibitory activity determined in (2) to select an immunosuppressive agent with a less thrombocytopenia effect.
<4> A method for selecting an immunosuppressive agent with a less thrombocytopenia effect, the method comprising the following items (1) to (3):
   (1) measuring an IL-2 transcription inhibitory activity in a test cell into which an IL-2 reporter gene has been introduced in the coexistence of the test cell and an analyte;
   (2) measuring a GATA-1 transcription inhibitory activity in a test cell into which a GATA-1 reporter gene has been introduced in the coexistence of the test cell and an analyte; and
   (3) comparing the IL-2 transcription inhibitory activity determined in (1) with the GATA-1 transcription inhibitory activity determined in (2) to select an immunosuppressive agent with a less thrombocytopenia effect.
<5> A method as described in <4> for selecting an immunosuppressive agent with a less thrombocytopenia effect, comprisingmeasuring a (IL-2 IC50) value as an IL-2 transcription inhibitory activity, measuring a (GATA-1 IC50) value as a GATA-1 transcription inhibitory activity, and comparing both the values.
<6> A method as described in <5> for selecting an immunosuppressive agent with a less thrombocytopenia effect, comprising selecting a compound having the (GATA-1 IC50) / (I1-2 IC50) value of 5 or more.
<7> A method as described in any of <4> to <6>, wherein the GATA-1 reporter gene comprises the transcriptional control region of human GATA-1 gene and a reporter gene.
<8> A method as described in any of <4> to <7>, wherein the GATA-1 reporter gene comprises a sequence of the region from -3769 to -3133 upstream of the transcription initiation point and sequence of the region from -789 to +30 proximal to the transcription initiation point of human GATA-1 gene.
<9> A method as described in any of <4> to <8>, wherein the IL-2 reporter gene comprises the transcriptional control region of IL-2 gene and a reporter gene.
<10> A method as described in any of <4> to <9>, wherein the IL-2 reporter gene comprises a sequence of the region from -378 to +54 proximal to the transcription initiation point of human IL-2 gene.
<11> A method as described in any of <4> to <10>, wherein the test cell into which a GATA-1 reporter gene is introduced is a human megakaryocytic cell strain.
<12> A method as described in any of <4> to <11>, wherein the test cell into which an IL-2 reporter gene is introduced is a human T cell-derived cell strain stimulated by phorbol 12-myristate 13-acetate, ionomycin and anti-CD28 antibody, and the test cell into which a GATA-1 reporter gene is introduced is a human megakaryocytic cell strain.
<13> A method as described in Claim 12, wherein the human T cell-derived cell strain is a Jurkat cell.
<14> A method as described in <11>, wherein the human megakaryocytic cell strain is a HEL cell.
<15> A method as described in <12>, wherein the human megakaryocytic cell strain is a HEL cell.
<16> A method as described in any of <4> to <15>, wherein the reporter gene is a firefly luciferase gene.
<17> A method for selection as described in any of <1> to <16>, wherein the analyte is an HDAC inhibitor.
<18> A method for selecting an immunosuppressive agent with a less thrombocytopenia effect, comprising measuring the amount of expression of GATA-1 protein.
<19> A method for selecting an immunosuppressive agent with a less thrombocytopenia effect, the method comprising the following items (1) to (3):
   (1) measuring the amount of expression of IL-2 protein;
   (2) measuring the amount of expression of GATA-1 protein; and
   (3) comparing both the amounts of expression to select an immunosuppressive agent with a less thrombocytopenia effect.
<20> A kit for measurement in selecting an immunosuppressive agent with a less thrombocytopenia effect, comprising the following items (1) and (2):
   (1) a DNA construct containing a GATA-1 reporter gene; and
   (2) a test cell of megakaryocytic cell line.
<21> A kit for measurement in selecting an immunosuppressive agent with a less thrombocytopenia effect, comprising the following items (1) to (4):
   (1) a DNA construct containing an IL-2 reporter gene;
   (2) a DNA construct containing a GATA-1 reporter gene;
   (3) a test cell of T-cell line; and
   (4) a test cell of megakaryocytic cell line.
<22> An HDAC inhibitor with a less thrombocytopenia effect, the inhibitor being selected by a method as described in <17>.
<23> An immunosuppressive agent with a less thrombocytopenia effect, the agent being selected by a method for selection as described in any of <1> to <16>.
<24> An immunosuppressive agent for treatment of inflammatory disorders, diabetes mellitus, diabetic complications, homozygous thalassemia, fibrosis, cirrhosis, acute promyelocytic leukemia, protozoal infections, organ transplant rejection, autoimmune diseases, and tumors, the agent comprising as an active ingredient an HDAC inhibitor as described in <22>.
<25> A therapeutic agent for treatment of inflammatory disorders, diabetes mellitus, diabetic complications, homozygous thalassemia, fibrosis, cirrhosis, acute promyelocytic leukemia, protozoal infections, organ transplant rejection, autoimmune diseases, and tumors, the agent comprising as an active ingredient an immunosuppressive agent as described in <23>.
<26> A therapeutic method for treatment of inflammatory disorders, diabetes mellitus, diabetic complications, homozygous thalassemia, fibrosis, cirrhosis, acute promyelocytic leukemia, protozoal infections, organ transplant rejection, autoimmune diseases, and tumors, the method comprising administering an immunosuppressive agent with a less thrombocytopenia effect containing as an active ingredient an HDAC inhibitor as described in <22>.
<27> A therapeutic method for treatment of inflammatory disorders, diabetes mellitus, diabetic complications, homozygous thalassemia, fibrosis, cirrhosis, acute promyelocytic leukemia, protozoal infections, organ transplant rejection, autoimmune diseases, and tumors, the method comprising administering an immunosuppressive agent with a less thrombocytopenia effect containing as an active ingredient an immunosuppressive agent as described in <23>.
<28> Use of an HDAC inhibitor as described in <22> in manufacture of a therapeutic agent for treatment of inflammatory disorders, diabetes mellitus, diabetic complications, homozygous thalassemia, fibrosis, cirrhosis, acute promyelocytic leukemia, protozoal infections, organ transplant rejection, autoimmune diseases, and tumors.
<29> Use of an immunosuppressive agent as described in <23> in manufacture of a therapeutic agent for treatment of inflammatory disorders, diabetes mellitus, diabetic complications, homozygous thalassemia, fibrosis, cirrhosis, acute promyelocytic leukemia, protozoal infections, organ transplant rejection, autoimmune diseases, and tumors.

The invention will be explained in detail as follows.

The invention relates to a method for selecting an immunosuppressive agent with a less thrombocytopenia effect, the method comprising measuring an IL-2 transcription inhibitory activity in a test cell into which an IL-2 reporter gene has been introduced in the coexistence of an analyte, while measuring a GATA-1 transcription inhibitory activity in the test cell into which a GATA-1 reporter gene has been introduced in the coexistence of an analyte, and comparing both the transcription inhibitory activities.

The term "IL-2 reporter gene" refers to a DNA construct in which a transcriptional control region of IL-2 gene is ligated artificially to a reporter gene. As the transcriptional control region of IL-2 gene, for example, a sequence comprising a proximal region of the transcription initiation point of IL-2 gene (GenBank Accession Number: X00695, Locus code: HSIL05) and its upstream region may be used. As the scope of the transcriptional control region necessary for the purpose of the invention, it is desired to not only hold a transcriptional activity but also substantially reflect the transcriptional control mode of a natural IL-2 gene in an active-type T cell. In fact, it has been reported that, in a human IL-2 gene, if a region of about 275 base pairs is present at the position upstream of the transcription initiation point, then it would function as a promoter reflecting substantially the transcriptional control mode of a natural IL-2 gene in a cell strain Jurkat (JCRB0062, Japanese Collection of Research Bioresources) derived from human T-lymphoblast (D.B. Durand et al., (1987) J. Exp. Med. 165, 395-407). From the studies to date, it has been found that the binding site of proteins such as NF-AT, OCT-1 (octamer-binding transcription factor-1), NF-κB, AP-1, CD28RC (CD28 responsive element binding complex), ZEB (TCF8, NIL-2-A zinc finger protein), and the like, is placed in this region. Therefore, if the region contains an essential region for such protein binding, any length of sequence could be used as the region for use in construction of an IL-2 reporter gene; for example, the region of 434 base pairs as described in Sequence Listing 9 (when the major transcription initiation point is +1, the region corresponds to from -378 to +54) may be used.

In order to establish a screening system for immunosuppressive agents effective to human, it is desired to use a transcriptional control region of an IL-2 gene of human origin. For the screening of compounds effective in a model animal such as mouse or rat, the transcriptional control region of IL-2 gene derived from each animal may be used, and such a DNA construct can easily be constructed by a person skilled in the usual experimental technique.

In the above-mentioned invention, the term "GATA-1 reporter gene" refers to a DNA construct in which a transcriptional control region of GATA-1 gene is ligated artificially to a reporter gene. As the transcriptional control region of GATA-1 gene, for example, a DNA fragment comprising both an IE promoter region of GATA-1 gene (GenBank Accession Number: AF196971) and an HSI region (highly sensitive region to DNase I) at about 3.5 kb separated from its upstream region maybe used. As the scope of the transcriptional control region necessary for the purpose of the invention, it is desired to not only merely hold a transcriptional activity but also substantially reflect the transcriptional control mode of a natural GATA-1 gene in megakaryocytic cells.

As mentioned above, it is known that as a promoter of GATA-1 gene there are at least 2 kinds of promoters, i.e., IE promoter and IT promoter. Among them, IE promoter is known to have a close relation with the transcriptional control in the megakaryocytic cells in relation with a thrombocytopenia effect by an HDAC inhibitor; thus, the function of IE promoter is estimated to be important, and its control region is utilized in this invention.

For the transcriptional control by IE promoter, a sequence present within 0.7 kb just upstream of the transcription initiation point and the further upstream HSI region (highly sensitive region to DNase I) both are important. There are 2 CACC sites at 5' end of the HSI region, and at the position separated about 50 base pairs from the HSI region, there are a GATA binding site and an E-box motif side by side and separated about 10 base pairs from each other. It has been known that when a mutation is introduced so as to destroy the GATA binding site in this situation, no expression specific to erythrocytes or megakaryocytes can be observed (P. Vyas et al., (1999) Development 126, 2799-2811). In addition, at the 3' end of GATA-E-box motif, a characteristic palindrome sequence CTGTGGCCACAG sequence and a region rich in GC are present. In the region rich in GC, a GGAA sequence seen in the ETS binding site of a transcriptional factor is present. In the GATA-1 HSI region containing these sequences, even though the 5'-side CACC site is deleted, the expression specific to erythrocytes or megakaryocytes can be observed, but when the GATE-E-box motif is deleted, no expression is observed. At the 3' end, it has been reported that those containing at most about 250 base pairs at downstream of E-box function as an enhancer. Therefore, if all of these essential sequence sites are involved, any length of the sequence might be accepted practically; for example, it is possible to use a sequence in which a region containing 819 base pairs proximal to the transcription initiation point (from -789 to +30; the transcription initiation point is fixed as +1) by IEpromoter of human GATA-1 gene is ligated artificially to a region containing 637 base pairs (from -3769 to -3133) as an HIS region at upstream of the transcription initiation point.

Regarding an essential sequence site such as GATE-E box motif, it is possible to prepare an artificial DNA construct in which a multiple of the essential sequences are linked together in tandem by a conventional recombinant DNA experimental technique. When such an artificial construct is used in place of a natural sequence, the transcriptional induction activity in the transcriptional control region can sometimes be increased.

In order to confirm a relationship between the GATE-1 transcription inhibitory activity of various HDAC inhibitors and the thrombocytopenia effect in an animal model such as mouse or rat, a similar DNA construct may be prepared using the transcriptional control region of GATA-1 gene of animal origin in place of the above-mentioned human sequence; such a DNA construct can easily be constructed by a person skilled in the usual experimental technique.

The term "reporter gene" as used in the invention refers to a gene which comprises a structural gene region coding for a specific protein as a marker of the gene expression and a downstream 3'-non coding region and in which the transcriptional control regions originally present at the upstream of the structural genes are deleted all. As for the structural gene region which codes for a specific protein serving as a marker of the gene expression, any type of ones may be used as far as the gene expression from the transcriptional control region contained in an extraneous gene fragment can easily be measured from the function of a marker protein corded by the reporter gene when it is artificially ligated to the extraneous gene fragment and introduced into a cell. More preferably, it is to be desirable that the protein as a marker can exist stably in mammal cells and no intrinsic protein having a similar activity exists in the cell or it can easily be distinguished from the marker even though it exists in the cell. More preferably, it is to be desirable that mRNA coding for a marker protein exists stably in cells. It is also desirable that in measurement of the activity, the substrate for the marker protein is not required to be added additionally or easily incorporated sufficiently in the cells even if addition is required. Further, the measurement system composed of these elements is desired to provide linearity in a wide range and high sensitivity. Luciferase gene of firefly origin, luciferase gene of Renilla origin, alkaline phosphatase, β-galactosidase gene, green fluorescent protein (GFP) gene, enhanced green fluorescent protein (EGFP) gene, β-glucuronidase gene, chloramphenicol acetyltransferase (CAT) gene, horse radish peroxidase (HRP) gene, and the like may be used. More preferably, luciferase gene of firefly origin may be used. As for luciferase gene of firefly origin, an improved type of luciferase gene (luc+) may be used to increase the detection sensitivity of a measurement system. For the non-coding region downstream of the structural gene, for example, a sequence derived from SV40 virus genome late gene may be used.

The above-mentioned IL-2 reporter gene and GATA-1 reporter gene are respectively ligated to a cloning vector and multiplied in Escherichia coli as a host. As a cloning vector used in this operation, a vector having the origin of replication and selective marker amplifiable in Escherichia coli can be used without any limitation; for example, pGL3-Basic (Promega Corporation) coding for an ampicillin resistance marker and an improved lucifurase structural gene (luc+) may be used to easily prepare the above-mentioned DNA construct. Thereafter, the amplified vector is introduced transiently into an animal cell to measure the reporter activity.

The DNA construct containing a IL-2 reporter gene and GATA-1 reporter gene maybe introduced by means of transformation utilizing any one of a usual calcium phosphate method, liposome method, lipofectin method, and electroporation method, without any limitation. More preferably, electroporation may be used.

As for the "test cell" into which an IL-2 reporter gene is introduced as a system for selecting immunosuppressive compounds, Jurkat cells (JCRB0062, Japanese Collection of Research Bioresources) may be used as a host cell, though other cell lines may be used as far as they are those derived from T cells. From the studies of the action mechanism of immunosuppressive agents such as cyclosporin A, tacrolimus, and the like, it has been known that the inhibition of the IL-2 expression in activated T-cells correlates well with the suppressive effect of these drugs for acute rejection after organ transplantation. Therefore, it is necessary to use as a test cell a cell substantially reflecting the state of human T cell during activation but not resting phase, in establishing a system for evaluating an immunosuppressive effect of ananalyte using an IL-2 reporter gene. In T cells, 2 signals are known, i.e., the first signal which mediates a TCR/CD3 complex recognizing a MHC complex representing CD4/CD8 and an antigenic peptide on the target cell, and the second signal which mediates CD28 recognizing CD80 or CD86 on the target cell. In order to maintain the activation of T cells, it is necessary to give stimulation so as to generate both of the above 2 signals at the same time, or give stimulation bypassing them. The non-specific activation of T cells caused by the first signal is known to be accompanied by phosphorylation of a group of proteins or increase of an intracellular calcium concentration; this effect empirically can be substituted by addition of a phorbol ester such as 12-myristate 13-acetate, or ionomycin. In addition, anti-CD28 antibody may be used as stimulation to the above second signal. The activated test cells used in the invention, accordingly, can be obtained by simultaneous stimulation of the above-mentioned host cells with phorbol 12 myristate 13 acetate, ionomycin or anti-CD28 antibody.

Similarly, as a "test cell" into which a GATA-1 reporter gene is introduced in selecting a compound with a less thrombocytopenia effect, it is necessary to use as a host cell a cell strain substantially reflecting the state of the cell of megakaryocytic cell line. As the cell line of megakaryocytes, for example, HELcell (JCRB0062, Japanese Collection of Research Bioresources) of human origin can be used without particular limitation.

The "analyte" as used in the invention means a candidate substance to be measured in the invention, including, but not limited to, low molecular organic compounds, low molecular inorganic compounds, high molecular compounds including proteins and nucleic acids, sugars, and all other compounds, as well as their liquid mixtures, natural products, synthetic products, and extracts from animals or fungi, algae, or microorganisms.

In the invention as mentioned above, "the coexistence of the test cell and an analyte" may be achieved by adding an analyte to a culture medium before or after introduction of an IL-2 reporter gene or GATA-1 reporter gene into a test cell by electroporation. More preferably, about 1 to 24 hours after introduction of an IL-2 reporter gene or GATA-1 reporter gene, more preferably about 3 to 12 hours after the introduction, an analyte may be added to the culture medium, and cultured for an additional 1 - 24 hours, more preferably for 8 - 12 hours.

In the invention as mentioned above, "IL-2 transcription inhibitory activity" can be determined in the above-mentioned test cell into which an IL-2 reporter gene has been introduced, by comparing the activity of the reporter gene product in the coexistence of an analyte with the standard activity of the reporter gene product determined in the absence of an analyte. When the reporter gene product is a sufficiently stable protein, the activity of this gene product is expected to be roughly proportional to the transcriptional activity of the IL-2 gene.

Similarly, in the invention as mentioned above, "GATA-1 transcription inhibitory activity" can be determined in the above-mentioned test cell into which a GATA-1 reporter gene has been introduced, by comparing the activity of the reporter gene product in the coexistence of an analyte with the standard activity of the reporter gene product determined in the absence of an analyte.

According to the method as mentioned above, a test cell into which an IL-2 reporter gene has been introduced (e.g., Jarkat cell) and a test cell into which a GATA-1 reporter gene has been introduced (e.g., HEL cell) can be constructed, respectively. These cells respectively are allowed to coexist with a variety of analytes, and the potency of IL-2 transcription inhibitory activity and of GATA-1 transcription inhibitory activity can be detected and measured using expression of the respective reporter genes as indicator. In order to select an immunosuppressive agent with a less thrombocytopenia effect, a substance which specifically inhibits expression of IL-2 but does not inhibit expression of GATA-1 gene in a test cell system may be selected.

Practically, in screening an effective compound clinically, it is appropriate to evaluate the thrombocytopenia effect of each analyte in balance with the potency of an immunosuppressive effect; that is, the standard of evaluation is determined by examining how degree of thrombocytopenia effect is attained at an effective dose at which a certain level of immunosuppressive effect is recognized, when the analyte is administered to the living body. Practically, this can be calculated as follows. That is, using the above-mentioned 2 expression systems for the reporter genes, the amount of expression of luciferase is determined. The term "IL-2 IC50" as used in the invention indicates the concentration of the added analyte at which the expression of the IL-2 reporter gene is inhibited by 50% when the amount of expression of IL-2 reporter gene is regarded as 100% where no analyte is added at all. Thus, IC50 can easily be calculated from a drug dose-response curve. This value is expected to correlate with an effective dose at which a drug shows a certain immunosuppressive effect *in vivo,* that is, a minimum effective dose at which the drug shows an immunosuppressive effect. Similarly, the term "GATA-1 IC50" as used in the invention indicates the concentration of the added analyte at which the expression of the GATA-1 reporter gene is inhibited by 50% when the amount of expression of GATA-1 reporter gene is regarded as 100% where no analyte is added at all. Thus, GATA-1 IC50 can easily be calculated from a drug dose-response curve. Thus calculated GATA-1 IC50 value is divided by the IL-2 IC50 value (i.e., (GATA-1 IC50) / (IL-2 IC50)); the resulting (GATA-1 IC50)/(IL-2 IC50) value is expected to correlate with the degree of thrombocytopenia effect caused by an analyte at a dose at which the analyte can show a certain degree of immunosuppressive effect when administered to a living body. That is, this (GATA-1 IC50)/(IL-2 IC50) value, for example, is expected to correlate with the potency of thrombocytopenia effect at the minimum effective dose of an analyte at which dose an immunosuppressive effect is recognized when administered to a living body. Practically, the (GATA-1 IC50)/(IL-2 IC50) value of each analyte obtained as mentioned above based on the reporter assay data, has been confirmed to well correlate with the rate of platelet decrease at a minimum dose at which the analyte administered to a rat's heart transplant model shows an immunosuppressive effect. In addition, it has been found that, for example, in a compound of which the (GATA-1 IC50)/(IL-2 IC50) value is 5 or higher, the rate of platelet decrease is suppressed to 30% or lower at a minimum dose at which an immunosuppressive effect is shown in a rat's heart transplant model.

As mentioned above, it is desirous to determine the "GATA-1 transcription inhibitory activity" and "IL-2 transcription inhibitory activity" using the activity of the reporter gene product as an indicator, by introducing an artificially constructed GATA-1 reporter gene or IL-2 reporter gene into a cell. Alternatively, it is also possible to measure the transcriptional activity by determining GATA-1 mRNA or IL-2 mRNA by means of RT-PCR or DNA microarray on the cell into which the GATA-1 gene or IL-2 gene containing all of the above-mentioned transcriptional control region *per se* has been introduced. Depending on some cell lines, it is also possible to directly monitor the expression of native GATA-1 gene or IL-2 gene endogenous in cells by means of RT-PCR or DNAmicroarray. Such an assay system can easily be established ingeniously by a person skillful in an experimental technique.

In addition, the invention relates to a method for selecting an immunosuppressive agent with less thrombocytopenia effect, the method comprising measuring the amount of expression of IL-2 protein, measuring the amount of expression of GATA-1 protein, and comparing both the amounts of expression.

In the invention as mentioned above, the "amount of expression of IL-2 protein" can be determined by treating an extract of the above-mentioned test cells containing no IL-2 reporter gene with an analyte, and measuring the amount of expression of IL-2 protein with an anti-IL-2 antibody and a labeled secondary antibody. As such a technique, a radioactive isotopic immunoassay (RIA), ELISA (E. Engvall et al., (1980), Methods in Enzymol., 70, 419-439), fluorescence antibody technique, plaque technique, spot method, aggregation method, Ouchterlony, and other various methods used in conventional immunochemical measurement techniques ("Hybridoma technique and monoclonal antibodies", published by R&D Planning, 30-53, March 5, (1982)) can be utilized. These techniques can easily be conducted by a person skillful in the art. Though the above-mentioned techniques can properly be chosen from many points of view, ELISA is preferably employed in view of sensitivity and convenience.

The labeling substance used in labeling the above secondary antibody includes enzymes such as horse radish peroxidase, alkaline phosphatase, etc., fluorescent substances such as fluorescein isocyanate, rhodamine, etc., radioactive substances such as ³²P, ¹²⁵I, etc., and chemiluminescences.

In the same technique, the "amount of expression of GATA-1 protein" may be determined by using an anti-GATA-1 antibody and a labeled secondary antibody.

In addition, the invention relates to a kit for measurement in selecting an immunosuppressive agent with a less thrombocytopenia effect, comprising a DNA construct containing an IL-2 reporter gene; a DNA construct containing a GATA-1 reporter gene; a test cell of T-cell line; and a test cell of megakaryocytic cell line.

In the invention as mentioned above, the "kit for measurement in selecting an immunosuppressive agent with a less thrombocytopenia effect" maybe used in measurement by detecting the potency of the IL-2 transcription inhibitory activity and of the GATA-1 transcription inhibitory activity in a test cell in the coexistence of the test cell into which an IL-2 reporter gene or GATA-1 gene has been introduced and an analyte, wherein the expression of IL-2 reporter gene or GATA-1 reporter gene is used as an indicator of the expression. Specifically, the kit comprises the following items (1) to (4):
(1) a DNA construct containing an IL-2 reporter gene;
(2) a DNA construct containing a GATA-1 reporter gene;
(3) a test cell of T-cell line, more preferably, Jurkat cell; and
(4) a test cell of megakaryocytic cell line, more preferably, HEL cell.

In the invention as mentioned above, the "DNA construct" refers to DNA containing the above-described IL-2 reporter gene or GATA-1 reporter gene, which may be or may not be incorporated in a cloning vector and may be cyclic or acyclic.

In addition, the invention relates to a therapeutic method and therapeutic agent for treatment of inflammatory disorders, diabetes mellitus, diabetic complications, homozygous thalassemia, fibrosis, cirrhasis, acute promyelocytic leukemia, protozoal infections, organ transplant rejection, autoimmune diseases, and tumors, comprising as an active ingredient a compounds which is obtained by measuring the (IL-2 IC50) value as an IL-2 transcription inhibitory activity, measuring the (GATA-1 IC50) value as a GATA-1 transcription inhibitory activity, and comparing both of the values to select a compound of which the (GATA-1 IC50)/(IL-2 IC50) value is 5 or higher.

According to the above-mentioned method for evaluation, the (IL-2 IC50) value is measured as an IL-2 transcription inhibitory activity, the (GATA-1 IC50) value is measured as a GATA-1 transcription inhibitory activity, and both of the values are compared to select a compound of which the (GATA-1 IC50) / (IL-2 IC50) value is 5 or higher; thus resulting compound is useful as an immunosuppressive agent with less thrombocytopenia effect in treatment or prevention of organ transplant rejection or autoimmune diseases. In addition, it is also useful in treatment or prevention of the following diseases possibly caused by abnormal gene expression: inflammatory disorders, diabetes mellitus, diabetic complications, homozygous thalassemia, fibrosis, cirrhosis, acute promyelocytic leukemia, protozoal infections, and tumors.

When these compounds are used as drugs in the invention, they can be used *per se* as drugs, or alternatively they may be formulated into pharmaceutical preparations according to a conventional pharmaceutical technology. For example, they may be properly combined with (a) pharmacologically acceptable conventional carrier(s) or vehicle(s), specifically sterilized water, physiological saline, vegetable oils, emulsifying agents, suspending agents, and the like, to form pharmaceutical preparations, for example, solid, semi-solid or liquid preparations (e.g., tablets, pills, troches, capsules, suppositories, cream, ointment, aerosol, powders, liquid preparations, emulsions, suspensions, and the like).

Administration to a patient may be achieved properly through nose, eye, external (local) site, rectum, lung (nose or oral injection), oral or parenteral (subcutaneous, intravenous or intramuscular) administration, or inhalation. Administration by injection may be achieved by a conventional method such as intra-arterial, intravenous, or subcutaneous injection.

The dosage of the compound of the invention as a therapeutic agent may be determined as a dose which shows a desired and satisfied therapeutic effect. The therapeutically effective dose of the compound, for example, for oral administration, is usually in about 0.1-100 mg, preferably 1-16 mg, a day. The effective single dose is chosen in the range of 0.001-1 mg, preferably 0.01-0.16 mg, for 1 kg of the patient weight. The above-mentioned dose, however, may be altered depending on the individual patient's body weight, age and condition, as well as method for administration. An appropriate dosage may be chosen properly by a person with a usual experimental technique based on animal experimental data.

### Brief Description of Drawings

Fig. 1 is a drawing showing that the platelet number in blood (n=5) is decreased depending on the dosage of an HDAC inhibitor, Compound B.
Fig. 2 is a drawing showing that the megakaryocyte number in spleen (n=5) is increased depending on the dosage of an HDAC inhibitor, Compound B.
Fig. 3 is a drawing showing that the amount of expressed GATA-1 (n=5) for unit megakaryocytes in spleen in which GPIIb has been measured as an internal standard is inhibited depending on the dosage of an HDAC inhibitor, Compound B.
Fig. 4 shows a map of plasmid pGL3 IL-2 Pro43.
Fig. 5 shows a map of plasmid pGL3-IE Promoter.
Fig. 6 shows a map of plasmid pGL3-HSI-IE Promoter.
Fig. 7 is a drawing showing that the luciferase activity in IL-2 reporter gene is inhibited depending on the concentration of Compound D.
Fig. 8 is a drawing showing that the cell growth in an IL-2 reporter gene assay system does not depend on the concentration of Compound D.
Fig. 9 is a drawing showing that the luciferase activity in GATA-1 reporter gene is inhibited depending on the concentration of Compound D.
Fig. 10 is a drawing showing that the cell growth in a GATA-1 reporter gene assay system does not depend on the concentration of Compound D.
Fig. 11 shows a correlation between the immunosuppressive effect and the thrombocytopenia effect and between the IL-2 and GATA-1 transcription inhibitory effects by an HDAC inhibitor in rats.
Fig. 12 is a drawing showing a pattern of the influence on transcription of a GATA-1 gene by 2 kinds of HDAC inhibitors (2 concentrations, respectively).
Fig. 13 shows a pattern of the influence on transcription of 45 genes selected from 12,626 genes by 2 kinds of HDAC inhibitors (2 concentrations, respectively), indicating that the transcription inhibitory pattern of these genes by HDAC inhibitors is similar to that of GATA-1 gene.
Fig. 14(a) shows the binding sequence of C/EBPβ used in C/EBPβ reporter plasmid.
Fig. 14(b) shows the binding sequence of HSF1 (or HSF2) used in an HSE reporter plasmid.
Fig. 14(c) shows the binding sequence of STAT3 used in a pSTAT3 reporter plasmid.
Fig. 15 shows the sequence of IE promoter (mutant). The capital letter indicates the site into which mutation is introduced.
Fig. 16 shows the sequence of HSI (mutant) region. The capital letter indicates the site into which mutation is introduced.

### Best Mode for Carrying Out the Invention

The invention will be explained more specifically by Examples, which are not intended as a limitation thereof. All of the prior technical literatures cited in the present specification are herein incorporated by reference.

### [Example 1] Confirmation of the thrombocytopenia effect caused by administration of an HDAC inhibitor

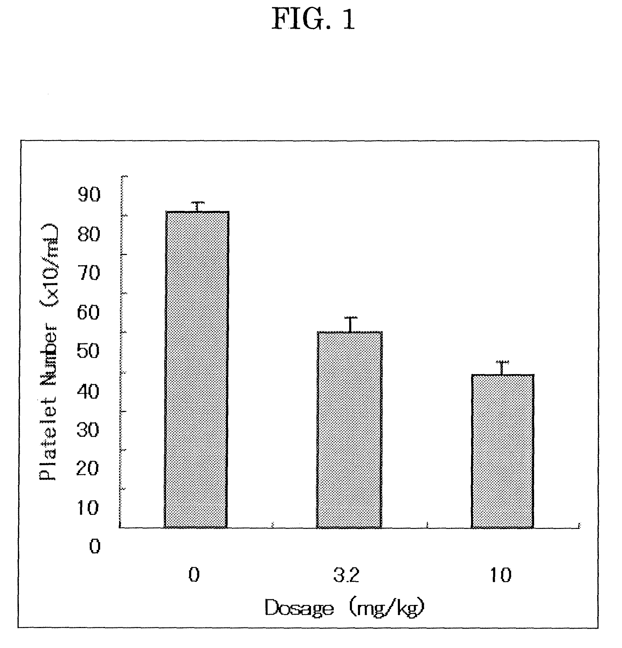

To male Lewis rats of 8 weeks of age was orally administered an HDAC inhibitor Compound B for 7 days. Rats were divided into 3 groups consisting of a 0 dose group, 3.2 mg/kg dose group, and 10 mg/kg dose group, wherein one group comprised 5 rats. Three hours after the final administration, blood was collected and the number of platelets was counted. Fig. 1 shows the number of platelets of each group thus counted. It was recognized that the platelet number was reduced depending on the dose of Compound B.

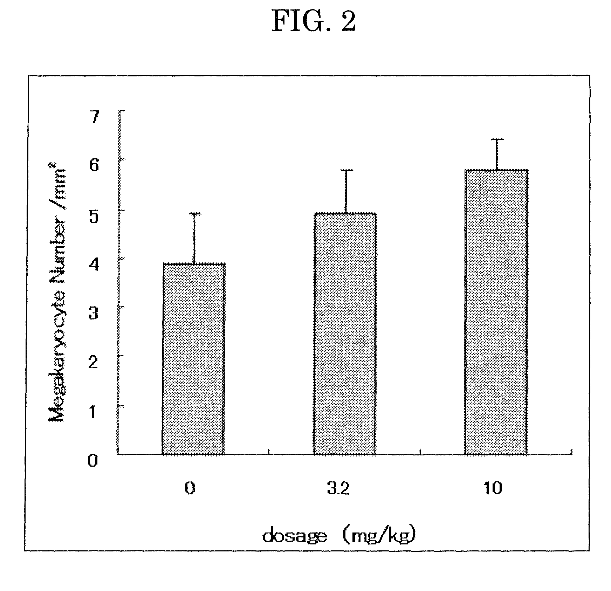

Subsequently, a sample of spleen was collected from the rats after drawing blood, and a part of the inferior margin was fixed with 4% paraformaldehyde (PFA) for counting the number of megakaryocytes to prepare aparaf fin section in a conventional way, which section was stained by HE (hematoxylin-eosin) staining. Each section was observed under an object lens of 10 magnifications to count the number of megakaryocytes/section in a bright-field. The number was divided by an area of the section to give the number of megakaryocytes in the spleen. Fig. 2 shows the average count of megakaryocytes in a group. It was recognized that the number of megakaryocytes in spleen was increased depending on the dose of Compound B.

### [Example 2] Determination of GATA-1 mRNA in the spleen of rats to which an HDAC inhibitor was administered

All the residual spleens after sampling in Example 1 were frozen and extracted with RNeasy (RNA extraction kit) to give total RNA as template, from which cDNA was synthesized by a Random Primer method. Subsequently, this cDNA was used as a template and the GATA-1 cDNA was amplified by a real-time PCR (SYBR technique) using ABI Prism 7700. The amount of GATA-1 mRNA was determined from its amplification curve.

In order to exactly determine the transcription amount of GATA-1 per megakaryocyte, it is necessary to use, as an internal standard for mRNA determination, a gene which is expressed specifically in megakaryocytes and of which the expression amount per megakaryocyte is not so influenced by administration of an HDAC inhibitor. Therefore, a mutation of gene expression influenced by an HDAC inhibitor in a cultured cell of megakaryocytic cell line, i.e., HEL cell, was analyzed using Gene Chip (Affymetrix), and glycoprotein IIb (GPIIb) was selected as an internal standard.

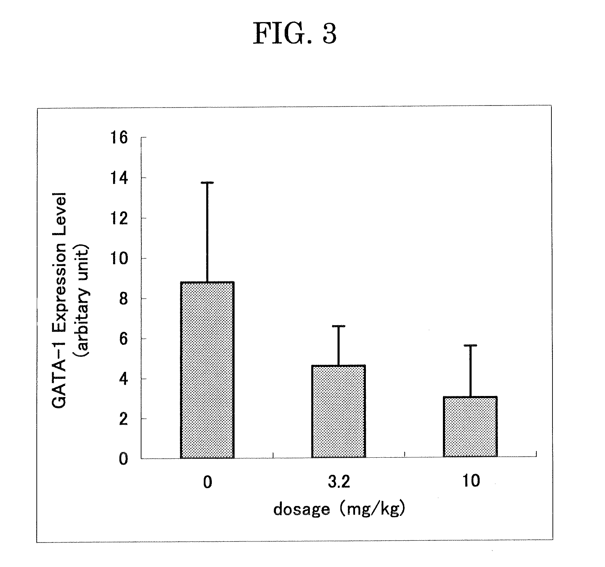

Fig. 3 indicates the amount of expressed GATA-1 determined using GPIIb as an internal standard. Inhibition of GATA-1 transcription was recognized to depend on the dose of an HDAC inhibitor Compound B. From the above experimental results, it was found that the inhibition of GATA-1 transcription was observed in rats in which the platelet was reduced by administration of an HDAC inhibitor.

### [Example 3] Construction of a reporter gene plasmid for IL-2 reporter gene assay

A DNA fragment proximal to the transcription initiation point of human IL-2 gene ranging from -674 to +54 (the main transcription initiation point of IL-2 gene is regarded as +1) was obtained by PCR using as a template a genomic DNA isolated from Jurkat cells of human T cell origin. The sequences of the primers used in PCR are shown in SEQ ID NOS: 1 and 2 in Sequence Listing. These primers were designed based on the IL-2 gene sequence (Locus code: HSIL05, Accession Number: X00695) as described in GenBank gene database, in which at the end of primers a restriction enzyme recognition site was added in order to introduce it into a vector for reporter gene assay. That is, the primers were designed so that a NheI recognition site was formed at the upstream side of the promoter of the amplified DNA fragment and a Hind III recognition site was formed at the downstream side of the promoter. The DNA fragment amplified by PCR was introduced into a cloning vector pCR4 (Invitrogen), and the base sequence of the insertion region was confirmed from the resulting plasmid. As a result, it was confirmed that theplasmidwas identical to the promoter sequence of IL-2 gene as described in the above GenBank, except for 3 sites of base substitution, 1 site of insertion of 2 bases, and 1 site of 1 base insertion. Total base sequence comprising 731 base pairs is shown in SEQ ID NO: 8. Thus resulting plasmid was cleaved at the Nhe I recognition site and Hind III recognition site, and the resulting fragment was inserted into the Nhe I-Hind III site of a vector pGL3 basic (Promega) for reporter gene assay containing a firefly luciferase gene. Thus, a plasmid pGL3 IL-2 Pro which has at the upstream of firefly luciferase gene a DNA sequence of 731 base pairs corresponding to the 728 base pair region proximal to the transcription initiation point of human IL-2 gene ranging from -674 to +54 was obtained.

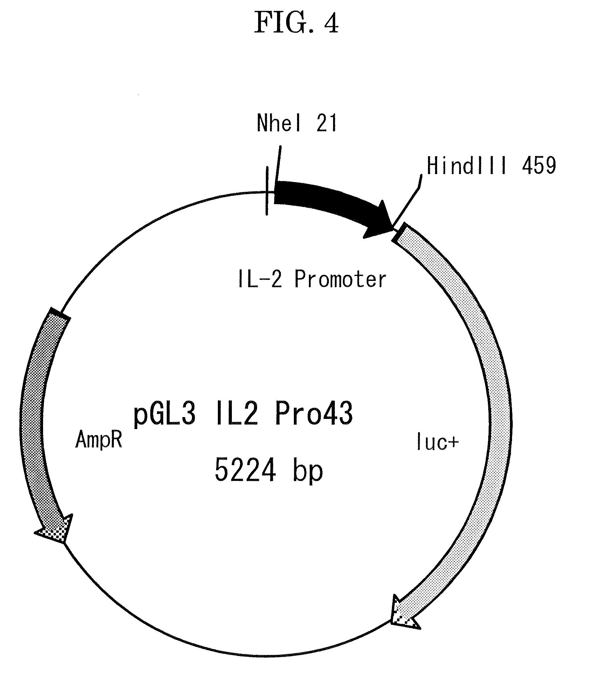

Subsequently, A DNA fragment proximal to the transcription initiation point of human IL-2 gene ranging from -378 to +54 was obtained by PCR using pGL3 IL-2 Pro as a template. The sequences of the primers used in PCR are shown in SEQ ID NOS: 3 and 2 in Sequence Listing. The primer of SEQ ID NO:3 were designed based on the DNA sequence introduced into the above-mentioned pGL3 IL-2 Pro. At the end of the primers a restriction enzyme recognition site was added in order to insert it into a vector for reporter gene assay. The primers are designed so that aNheI recognition site is formed at the upstream side of the promoter of the amplified DNA fragment and a Hind III recognition site was formed at the downstream side of the promoter. The DNA fragment amplified by PCR was inserted into a cloning vector pCR4, and the base sequence of the insertion region was confirmed using the resulting plasmid. This insertion region was identical to the region ranging from -378 to +54 of the IL-2 promoter sequence as described in GenBank Locus code: HSIL05, except for 2 sites of base substitution and 1 site of insertion of 2 bases. Total base sequence comprising 434 base pairs is shown in SEQ IDNO: 9. Thus resulting plasmid was cleaved at the Nhe I recognition site and Hind III recognition site, and the resulting fragment was inserted into the Nhe I-Hind III site of a vector pGL3 basic for reporter gene assay containing a firefly luciferase gene. Thus, a plasmid pGL3 IL-2 Pro43 which had at the upstream of firefly luciferase gene a promoter sequence of 434 base pairs corresponding to the 432 base pairs proximal to the transcription initiation point of human IL-2 gene ranging from -378 to +54 was obtained (Fig. 4).

### [Example 4] Construction of a reporter gene plasmid for GATA-1 Reporter Gene Assay

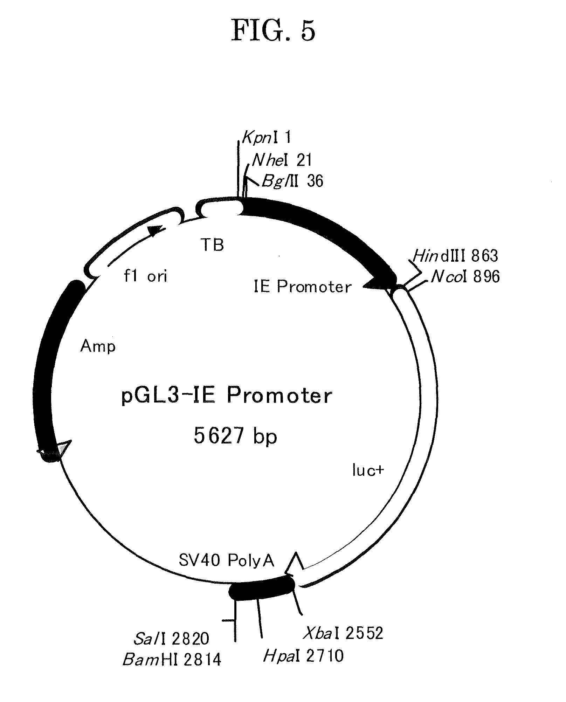

A fragment of human GATA-1 gene of 819 base pairs proximal to the transcription initiation point ranging from -789 to +30 was obtained by PCR using a human genome DNA as a template. In PCR, the primers as described in SEQ ID NOS: 4 and 5 in Sequence Listing were used. These primers were designed based on the GATA-1 gene sequence of Accession Number: AF196971 as described in GenBank gene database, in which at the end of primers a restriction enzyme recognition site was added in order to insert it into a vector for reporter gene assay. Thus, a Bgl II recognition site was formed at the upstream side of the promoter of the amplified DNA fragment and a Hind III recognition site was formed at the downstream side of the promoter. The DNA fragment amplified by PCR was introduced into a cloning vector pCR4. The base sequence of the insertion region in the resulting plasmid was confirmed to be fully identical with the GATA-1 promoter sequence as described in GenBank Accession Number: AF196971. The total base sequence is shown in SEQ ID NO: 10. Thus resulting plasmid was cleaved at the Nhe I recognition site and Hind III recognition site. The resulting fragment was inserted into the Nhe I-Hind III site of a vector pGL3 basic for reporter gene assay containing a firefly luciferase gene. Thus, a plasmid pGL3-IE Promoter which had at the upstream of firefly luciferase gene a sequence of 819 base pairs of GATA-1 gene promoter ranging from -789 to +30 was obtained (Fig. 5).

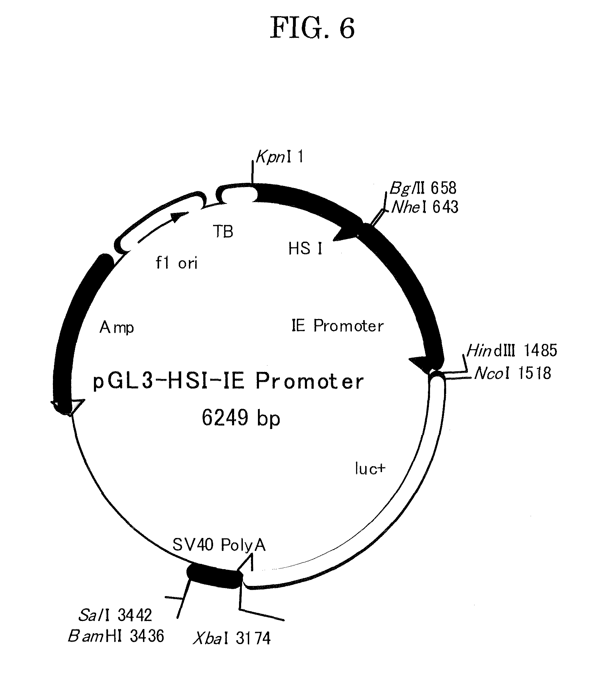

Subsequently, a fragment of 637 base pairs of human GATA-1 gene ranging from -3769 to -3133 at the upstream of transcription initiation point was prepared by PCR using a human chromosomal DNA as a template. In PCR, the primers as described in SEQ ID NO: 6 and 7 in Sequence Lisiting were used. These primers were designed based on the GATA-1 gene sequence of Accession Number: AF196971 as described in GenBank gene database, in which at the end of primers a restriction enzyme recognition site was added in order to insert it into a vector for reporter gene assay. Thus, a Kpn I recognition site was formed at the upstream side of the promoter of the amplified DNA fragment and a Nhe I recognition site was formed at the downstream side of the promoter. The DNA fragment amplified by PCR was inserted into a cloning vector pCR4, and the base sequence of the insertion region in the resulting plasmid was confirmed to be fully identical with the sequence at the upstream of transcription initiation point of the GATA-1 gene as described in GenBank AccessionNumber: AF196971. The total base sequence consisting of 637 base pairs is shown in SEQ ID NO: 11. Thus resulting plasmid was cleaved at the Kpn I recognition site and Nhe I recognition site, and the resulting DNA fragment was inserted into the Kpn I-Nhe I site of a vector pGL3 IE Promoter. Thus, a plasmid pGL3-HSI-IE Promoter which had at the upstream of firefly luciferase gene a region of 637 base pairs ranging from -3769 to -3133 and a sequence of 819 base pairs ranging from -789 to +30 at the upstream of transcription initiation point of the GATA-1 gene was obtained (Fig. 6).

### [Example 5] Construction of a screening system for an HDAC inhibitor having an IL-2 transcription inhibitory effect

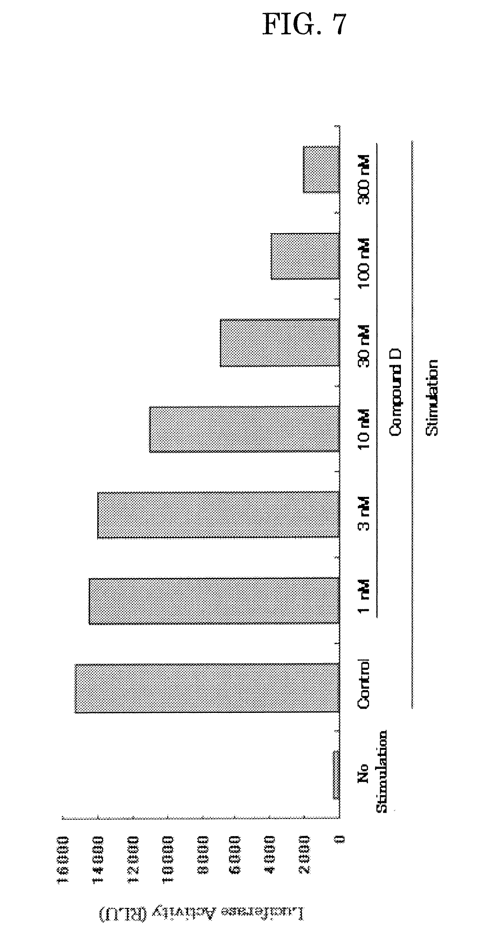

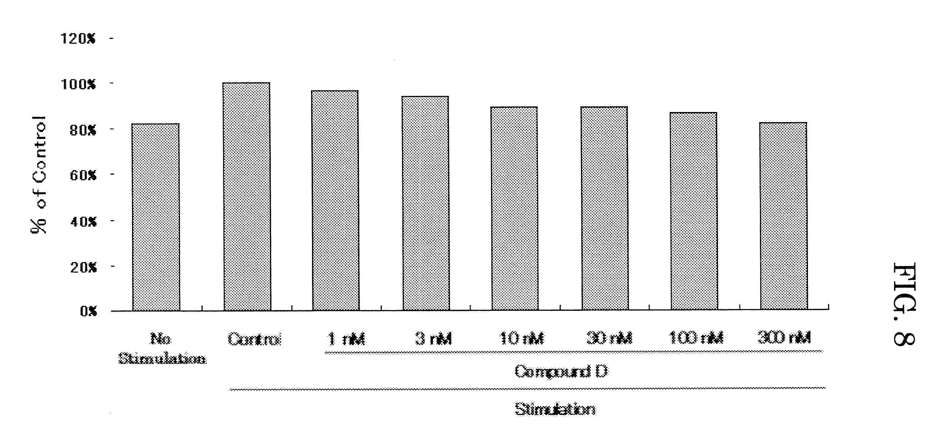

pGL3 IL-2 Pro43 (1µg) obtained in Example 3 was mixed with 6µg of optional carrier plasmid, and Jurkat cells (1x10⁷ cells) were transformed therewith by electroporation (Voltage 300V; charge 975µF; 400µL). After transformation, there was added 2.5 mL of 10% FBS-containing RPMI 1640 (10%FBS RPMI1640), which was distributed to a 96-well white plate at a rate of 50µl/well. The plate was incubated at 37°C under 5% CO₂ in a condition of saturated humidity for 12 hours, and then 10%FBS RPMI1640 medium containing Compound D (HDAC inhibitor) at a concentration of 4 times over the final concentration was added at a rate of 25µL/well. Additionally, a mixture of phorbol 12-myristate 13-acetate (PMA, SIGMA), ionomyicn (SIGMA) and anti-CD28 antibody (Pharmingen) in 10%FBS RPMI1640 was added at a rate of 25µL/well (final concentration, 50ng/mL, 1µg/mL and75ng/mL, respectively) tostimulatetheJurkatcells. After incubation at 37°C under 5% CO₂ in a condition of saturated humidity for 12 hours, the luciferase activity in the cells was measured with a multi-label counter (1420 MULTILABEL COUNTER ARVO SX, WALLAC) according to a manual of Bright-Glo™ Luciferase Assay System (Promega Corporation). As a result, it was found that luciferase was derived from pGL3 IL-2 Pro43 in response to stimulation of Jarkat cells with PMA, ionomycin and anti-CD28 antibody (Fig. 7). Further, it was found that the amount of expressed luciferase derived therefrom was inhibited dose-dependently by Compound D (Fig. 7). It was confirmed by Cell Counting Kit-8 (Dojindo Laboratories) that suppression of the amount of expressed luciferase by Compound D was not a result of some damage of the cells by Compound D (Fig. 8). From the above-mentioned results, it is confirmed that this assay system can be used in screening of an HDAC inhibitor having IL-2 transcription inhibitory effect.

### [Example 6] Construction of a screening system for an HDAC inhibitor Compound D having GATA-1 transcription inhibitory effect

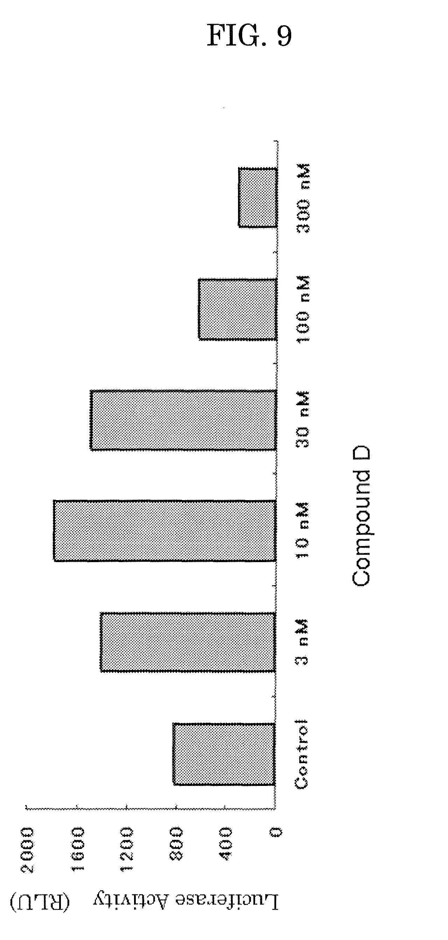

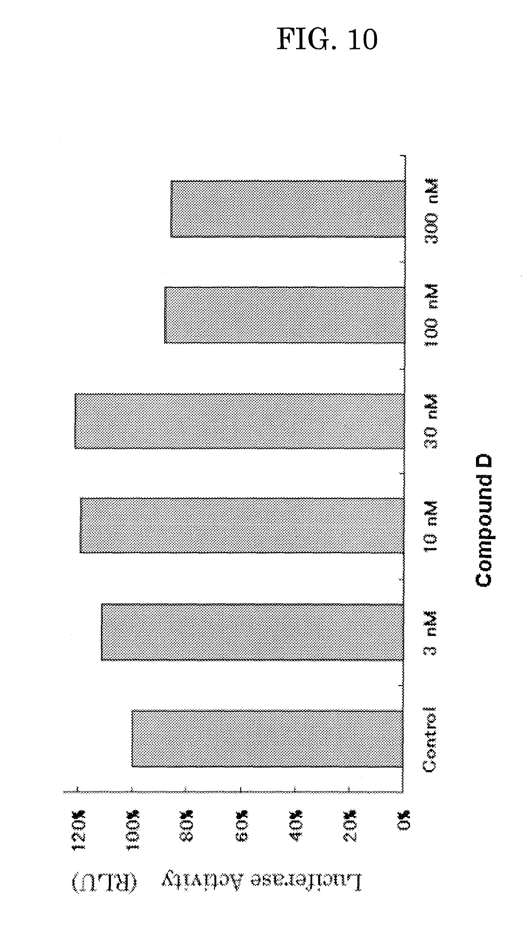

Using 15µg of pGL3-HSI-IE promoter obtained in Example 2, HEL cells (JCRB0062, Japanese Collection of Research Bioresources)(8.75x10⁶ cells) were transformed by electroporation (Voltage 1750V; charge 10µF; 365µL). After transformation, there was added 2 mL of 10%FBS RPMI1640, and the mixture was distributed to a 96-well white plate at a rate of 50 µL/well. After incubation at 37°C under 5% CO₂ in a condition of saturated humidity for 3 hours, 10%FBS RPMI1640 medium containing an HDAC inhibitor Compound D at a concentration of 2 times over the final concentration was added at a rate of 50µL/well. After incubation at 37°C under 5% CO₂ in a condition of saturated humidity for 8 hours, the luciferase activity in the cells was measured with a multi-label counter according to a manual of Bright-Glo™ Luciferase Assay System (Promega Corporation). As a result, it was found that luciferase was derived from pGL3-HSI-IE promoter depending on a GATA-1 promoter, and further the amount of expressed luciferase was inhibited dose-dependently by Compound D (Fig. 9). It was confirmed by Cell Counting Kit-8 (Dojindo Laboratories) that the inhibition of the amount of expressed luciferase by Compound D was not a result of some damage of the cells by Compound D (Fig. 10). From the above-mentioned results, it was confirmed that this assay system can be used in screening of an HDAC inhibitor having GATA-1 transcription inhibitory effect.

### [Example 7] Confirmation of the rate of platelet decrease in the minimum effective dose of an HDAC inhibitor with an immunosuppressive effect in rat's heart transplantation

Nine HDAC inhibitory compounds selected at random as analytes (as shown in Tables 1 and 2) were evaluated in an allopatric heart-to-neck transplant model (Cuff method), wherein ACI rats were used as donors, and Lewis rats as recipients. Each compound was orally administered with 10% HCO-60 (polyoxyl 60 hydrogenated castor oil)/water as solvent or subcutaneously with 10% HCO-60/saline as solvent, once a day continuously for 14 days from the day of transplantation. Cardiac arrest was judged as occurrence of rejection, and the number of days of take was regarded as up to the day before the cardiac arrest. Since the median of the number of take days is 5 days in a control group (solvent-administered group), the median over 8 days was judged effective, and the minimum effective dose was determined, respectively.

Next, the analyte was administered to normal Lewis rats at the minimum effective dose respectively determined above in the same administration route as in a pharmacological test once a day, totally 7 times. The day after the final administration, blood was collected, and the number of platelet was counted in individuals. Thus, the rate (%) of decrease to the control group was calculated from the mean value in each group (n=4).

Tables 1 and 2 indicate the minimum effective dose and the rate of platelet decrease thus obtained in a rat's heart transplant model with a variety of HDAC inhibitors. The dose judged as the minimum effective dose is indicated by an underline and boldface.

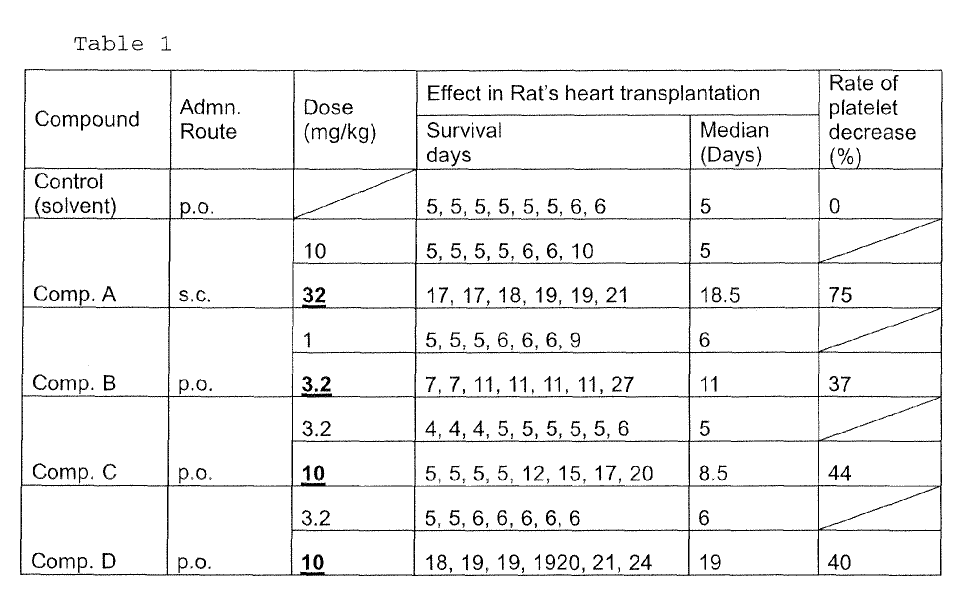

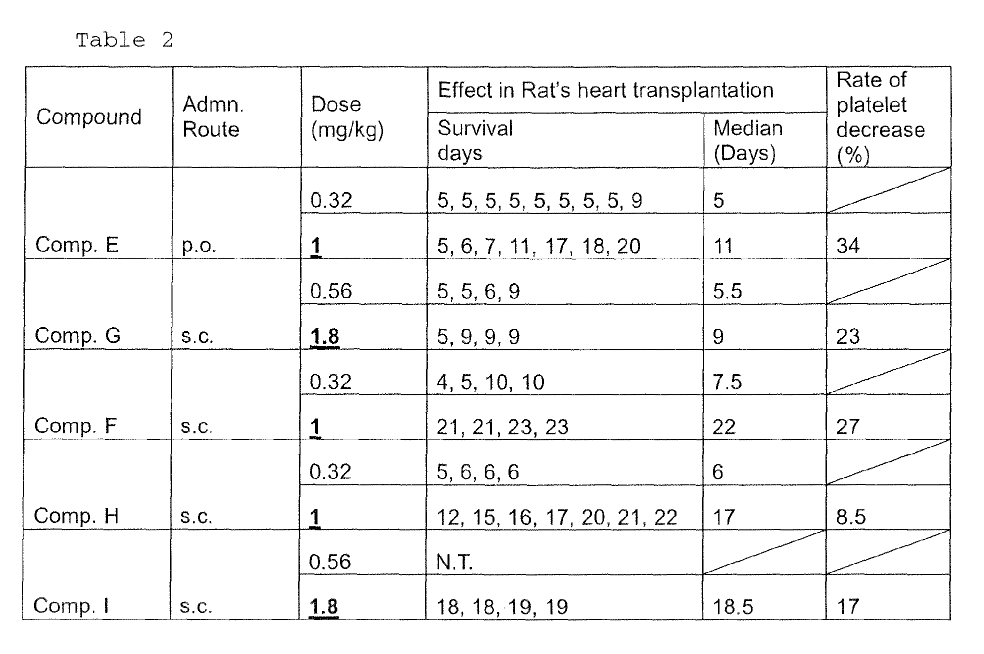

### [Example 8] Method for selecting an HDAC inhibitor which has an immunosuppressive effect with a less thrombocytopenia effect

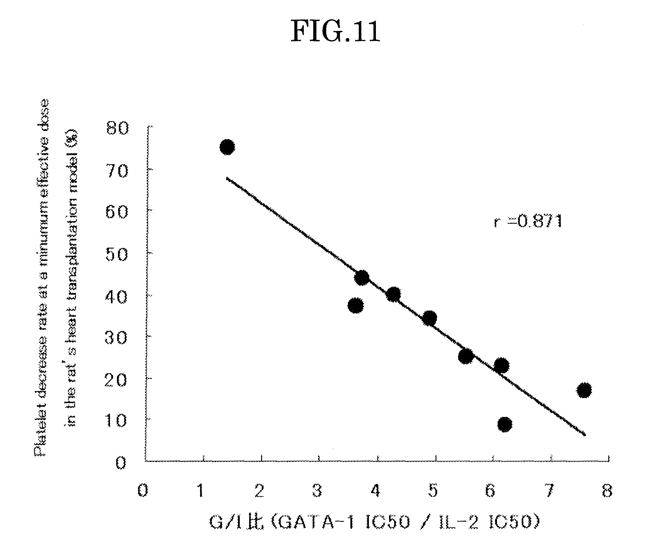

Compounds as shown in Table 3 were used as analytes for assay using a method for screening an HDAC inhibitor having an IL-2 transcription inhibitory effect as shown in Example 3. The concentration of a drug inhibiting by 50% of the expression of luciferase was calculated as the IC50 value (IL-2 IC50) for the inhibition of IL-2 transcription from a dose-response curve when the amount of expression of luciferase in the absence of an analyte was regarded as 100% (Table 3). Subsequently, the compounds as shown in Table 1 were used as analytes for assay using a method for screening an HDAC inhibitor having an GATA-1 transcription inhibitory effect as shown in Example 4. The concentration of a drug inhibiting by 50% of the expression of luciferase was calculated as the IC50 value (GATA-1 IC50) for the inhibition of GATA-1 transcription from a dose-response curve when the amount of expression of luciferase in the absence of an analyte was regarded as 100% (Table 3). Further, the G/I ratio was calculated by dividing GATA-1 IC50 by IL-2 IC50 (Table 3). The rate of platelet decrease at a minimum effective dose of an analyte in the rat's heart transplantation obtained in Example 5 and the G/I ratio obtained in this example were plotted (Fig. 11) to give a good correlation. In addition, it was found that the compounds showing a G/I ratio of 5 or more reduced the rate of platelet decrease to 30% or lower at a minimum effective dose at which the compounds showed an immunosuppressive effect in a rat's heart transplant model. From the above results, it was confirmed that this assay system is suitable for screening an HDAC inhibitor having an immune effect with a less thrombocytopenia effect.

**Table 3**

| HDAC Inhibitor HDAC Inhibitor | Reporter Gene Assay IC50 (nM) | | Ratio GATA-1/IL-2 |
|---|---|---|---|
| | IL-2 | GATA-1 | |
| Compound A | 342.9 | 475.7 | 1.39 |
| Compound B | 32.3 | 117.3 | 3.63 |
| Compound C | 8.7 | 32.6 | 3.73 |
| Compound D | 27.5 | 117.3 | 4.27 |
| Compound E | 30.7 | 150.5 | 4.90 |
| Compound F | 23.1 | 128.0 | 5.53 |
| Compound G | 14.8 | 91.0 | 6.16 |
| Compound H | 6.1 | 37.8 | 6.20 |
| Compound I | 19.6 | 148.6 | 7.58 |

### [Example 9] Search of a transcriptional factor relating to a thrombocytopenia effect by an HDAC inhibitor using a GeneChip analysis

From the genes expressed in megakaryocytes, a gene of which the expression is inhibited by an HDAC inhibitor in the same pattern as GATA-1 was screened and grouped with a GeneChip (Affymetrix) to search a transcriptional factor common to these gene groups.

Human megakaryocytic culture cells, HEL cells, were treated with 2 kinds of HDAC inhibitors showing a thrombocytopenia effect (500nM and 1500nM of Compound A, and 100nM and 300nM of Compound D) for 8 hours, and then extracted with RNeasy Kit (QIAGEN) to give the total RNA. The total RNA was used as a template to synthesize cDNA using a Superscript Choice System (Invitrogen) and further synthesize a fluorescence labeled cRNA using BioArray High Yield RNA transcript Labeling Kit (Enzo Diagnostics). The resulting fluorescence labeled cRNA was used as a probe to investigate the expression pattern of 12,626 genes using a Human Genome U95A Array (Affymetrix).

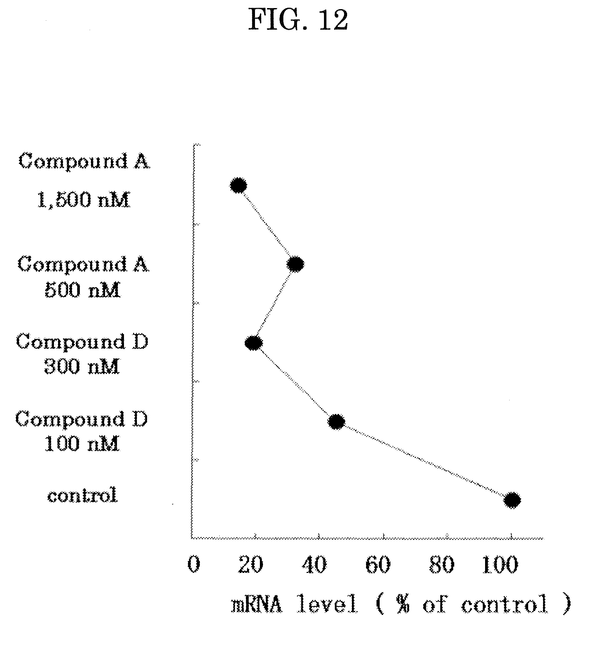

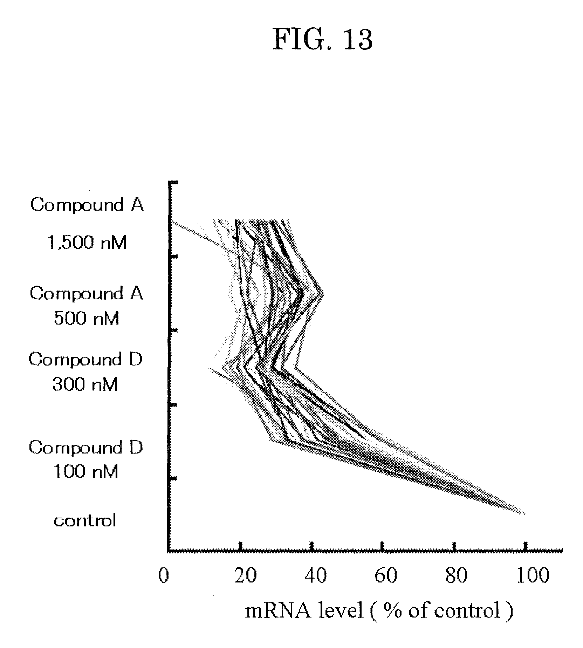

The effect of the above 2 HDAC inhibitors influencing the transcription of GATA-1 gene showed a pattern as shown in Fig. 12. This expression pattern was comparatively analyzed by a gene expression analyzing tool, GeneSpring (Silicon Genetics). As a result, 45 types of genes was found, of which the transcription was inhibited by the HDAC inhibitors in the same pattern as the GATA-1 gene (Fig. 13). From these genes, further, candidate genes as transcriptional factor expected to have a function associated with differentiation of blood corpuscles or candidate genes expected as megakaryocyte marker genes were selected, and the following 10 genes were obtained.
SCL (GenBank Accession Number M63589: P.D. Aplan et al., (1990) Mol. Cell. Biol. 10(12), 6426-6435)
NF-E2 (GenBank Accession Number S77763: C. Pischedda et al., (1995) Proc. Natl. Acad. Sci. U.S.A. 92(8), 3511-3515)
EKLF (GenBank Accession Number U65404: J.H. van Ree et al., (1997) Genomics 39(3), 393-395)
Pleckstrin (GenBank Accession Number X07743: M. Tyers et al., (1988) Nature 333 (6172), 470-473)
Thrombin-R (GenBank Accession Number M62424: T.K. Vu et al., (1991) Cell 64(6), 1057-1068)
LMO2 (GenBank Accession Number X61118: B. Royer-Pokora et al., (1991) Oncogene 6(10), 1887-1893)
PU.1 (GenBank Accession Number X52056: D. Ray et al., (1990) Oncogene 5(5), 663-668)
Fli-1 (GenBank Accession Number M98833: D.K. Watson et al., (1992) Cell Growth Differ. 3(10), 705-713)
AML1 (GenBank Accession Number D43968: H. Miyoshi et al., (1991) Proc. Natl. Acad. Sci. U.S.A. 88(23), 10431-10434)
TCF11 (GenBank Accession Number L24123 : J.Y. Chan et al., (1993) Proc. Natl. Acad. Sci. U.S.A. 90(23), 11371-11375)

Subsequently, the sequence information on the 2kb upstream of transcription initiation sites of the above-mentioned respective genes was downloaded from the human genome sequence information (Genome Annotation, NCBI Locus Link), and the database of transcriptional factors (The Transcription Factor Database, TRANSFAC^{(R)} Professional 6.3, BIOBASE Biological Databases /Biologische Datenbanken GmbH) was searched based on a prediction assisting system for transcriptional control regions TRANSFAC (E. Wingender et al., (2000) Nucleic Acids Research 28, 316-319, TRANSFAC: an integrated system for gene expression regulation) to investigate the transcriptional factor binding sequences commonly present in the respective promoter sequences of human origin. In this procedure, in the transcriptional control region within 2kb upstream of the transcription initiation point of the above-mentioned respective genes of which the expression was inhibited by an HDAC inhibitor, the transcriptional factor binding sequences commonly present in almost of genes, more particularly, the sequences commonly present in either of IE promoter and HSI region in GATA-1 gene, were mainly selected. As a result, the following 4 sequences (1) to (4) were found as candidates of the transcriptional factors involved in the inhibition of expression of the above-described genes by an HDAC inhibitor.
(1) STAT3: signal transducer and activator of transcription 3 (acute-phase response factor)
(2) C/EBPβ: CCAAT/enhancer binding protein (C/EBP), beta
(3) HSF1 (or HSF2): heat shock transcription factor 1 (or heat shock transcription factor 2)
(4) GATA-1: GATA binding protein 1

### [Example 10] Construction of a reporter gene (luciferase) vector having a responsive sequence recognized by STAT3, C/EBPβ and HSF1

Among the 4 candidates of transcriptional factors, STAT3, C/EBPβ, HSF1 and GATA-1, involved in the transcriptional control of the GATA-1 gene by an HDAC inhibitor, for the 3 transcriptional factors, STAT3, C/EBPβ and HSF1 (or HSF2), a reporter gene (luciferase) vector having a responsive sequence recognized by the respective transcriptional factors was constructed.

### (C/EBPβ reporter plasmid)

CEBP-11 and CEBP-12 represented by the following sequences were designed, in which the so far reported 5 consensus sequences recognized by C/EBPβ (M. Rosati et al., (2001) The Journal of Immunology 167, 1654-1662, CCAAT-Enhancer-Binding Protein β (C/EBPβ) Activates CCR5 Promoter: Increased C/EBPβ and CCR5 in T Lymphocytes from HIV-1-Infected Individuals) (S. Osada et al., (1996) The Journal of Biological Chemistry 271, 3891-3896, DIVA Binding Specificity of the CCAAT/Enhancer Binding Protein Transcription Factor Family) were connected in series to give synthetic DNAs (Amersham Biosciences).

CEBP-11:5'-CGCGTTGAGCAAGACTTGAGCAAGTACTTGAGCAAGCGTTGAGCAAG
GCTTGAGCAAGC-3'

CEBP-12:5'-TCGAGCTTGCTCAAGCCTTGCTCAACGCTTGCTCAAGTACTTGCTCA

AGTCTTGCTCAA-3'

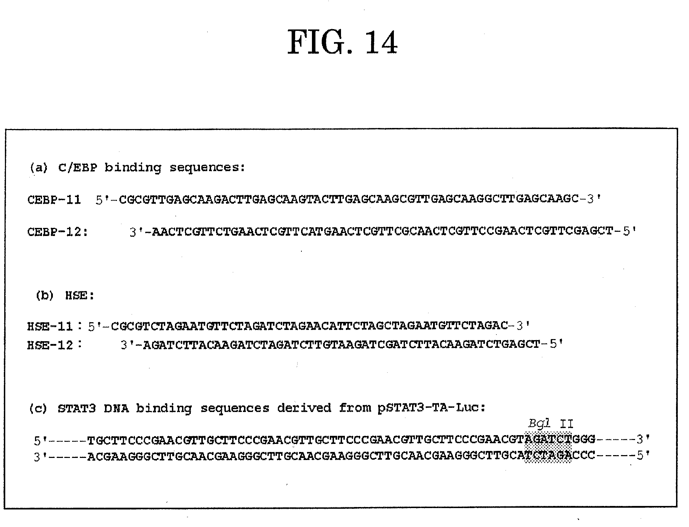

The above-described 2 synthetic DNAs were mixed and annealed to give a fragment of C/EBPβ binding sequence as shown in Fig. 14(a). This fragment was inserted into the multi-cloning site MluI/XhoI of pTA-Luc (BD Biosciences Clontech) having a TA minimal promoter to give pC/EBPβ-TA-Luc. On the other hand, pTAL-Luc (BDBiosciences Clontech) was treated with BglII/SphI to give a fragment of about 0.7kb coding for TAL promoter sequence and firefly luciferase gene N-terminal region. This fragment was ligated to a fragment of about 4.2kb prepared by treatment of pC/EBPβ-TA-Luc with BglII/SphI to give a C/EBPβ reporter plasmid pC/EBPβ-TAL-Luc which contains a TAL promoter sequence and firefly luciferase gene at the downstream of the C/EBPβ binding sequence.

### (HSE reporter plasmid)

According to the HSE (heat shock promoter element) used in pHSE-TAL-Luc (BD Biosciences Clontech), HSE-11 and HSE-12 as shown by the following sequences were designed to give synthetic DNAs (Amersham Biosciences).

HSE-11:5'-CGCGTCTAGAATGTTCTAGATCTAGAACATTCTAGCTAGAATGTTCTA

GAC-3'

HSE-12:5'-TCGAGTCTAGAACATTCTAGCTAGAATGTTCTAGATCTAGAACATTCT
AGA-3'

The above-described 2 synthetic DNAs were mixed and annealed to give a fragment of HSE sequence as shown in Fig. 14 (b). This fragment was ligated to a fragment of about 4.9kb prepared from pTA-Luc (BD Biosciences Clontech) by treatment with MluI/XhoI to give pHSE-TA-Luc. On the other hand, pTAL-Luc (BD Biosciences Clontech) was treated with BgIII/SphI to give a fragment of about 0.7kb coding for a TAL promoter sequence and firefly luciferase gene N-terminal region. This fragment was ligated to a fragment of about 4.2kb prepared by treatment of pHSE-TA-Luc with BglII/SphI to give a HSF1 reporter plasmid pHSE-TAL-Luc which contains a TAL promoter sequence and firefly luciferase gene at the downstream of the HSE sequence.

### (pSTAT3 reporter plasmid)

pTAL-Luc (BD Biosciences Clontech) was treated with BglII/SphI to give a fragment of about 0.7kb coding for TAL promoter sequence and firefly luciferase gene N-terminal region. This fragment was ligated to a fragment of about 4.2kb prepared from pSTAT3-TA-Luc (BD Biosciences Clontech) by treatment with BglII/SphI to give a STAT3 reporter plasmid pSTAT3 -TAL-Luc which contains a TAL promoter sequence and firefly luciferase gene at the downstream of the STAT3 binding sequence as shown in Fig. 14(c).

### [Example 11] Effect of an HDAC inhibitor on the transcriptional activity of STAT3, C/EBPβ and HSF1

HEL cells (8.75x10⁶ cells) were transformed with 15µg each of pC/EBPβ-TAL-LUc, pHSE-TAL-Luc, and pSTAT3-TA-Luc prepared in Example 10 by electroporation (Voltage 17 50V; Charge 10µF, 365µL). After transformation, the transformants respectively were suspended in 2 mL of 10% FBS RPMI1640, and distributed into a 96-well white plate at a rate of 50µL/well. The plate was incubated at 37°C under 5% CO₂ in a condition of saturated humidity for 3 hours, and then 10%FBS RPMI1640 medium containing Compound D (HDAC inhibitor) at a concentration of 2 times over the final concentration was added at a rate of 50µL/well. After incubation at 37°C under 5% CO₂ in a condition of saturated humidity for 8 hours, the luciferase activity in the cells was measured with a multi-label counter according to a manual of Bright-Glo™ Luciferase Assay System (Promega Corporation). As a result, it was found that luciferase is derived from pC/EBPβ-TAL-LUc, pHSE-TAL-Luc, and pSTAT3-TA-Luc depending on the transcriptional activity of C/EBPβ, HSF1 and STAT3, respectively. Their induction of luciferase activity wasnot inhibi ted by Compound D (Table 4). From the above results, it was elucidated that the transcriptional inhibitory effect of GATA-1 by an HDAC inhibitor is not due to the transcriptional inhibitory effect of C/EBPβ, HSF1 (or HSF2) and STAT3.

**Table 4**

| Compound D (nM) | Luciferase activity (%) | | |
|---|---|---|---|
| | pC/EBPβ-TAL-LUc | pHSE-TAL-Luc | pSTAT3-TAL-Luc |
| 0 | 100 | 100 | 100 |
| 3 | 173 ± 3 | 163 ± 7 | 166 ± 10 |
| 10 | 277 ± 4 | 258 ± 9 | 238 ± 7 |
| 30 | 367 ± 9 | 338 ± 18 | 316 ± 16 |
| 100 | 388 ± 5 | 355 ± 12 | 334 ± 18 |
| 300 | 379 ± 1 | 361 ± 3 | 326 ± 13 |
| Relative activity wherein the average luciferase activity is 100 with no addition of Compound D | | | |
| Mean ± Standard error (n=3) | | | |

### [Example 12] Construction of a reporter gene (luciferase) vector having a mutant promoter in which mutation is introduced into all of the GATA-1 recognition sites in the GATA-1 promoter

GATA-1 promoter has therein a responsive sequence (GATA sequence) recognized by GATA-1 *per se.* In order to elucidate whether the transcriptional control of the GATA-1 gene by an HDAC inhibitor is associated with the transcriptional control system through the GATA sequence, a promoter in which the responsive sequence recognized by GATA-1 *per se* was eliminated by introducing a mutation into the GATA sequence was artificially constructed and ligated to a reporter gene to give a vector.

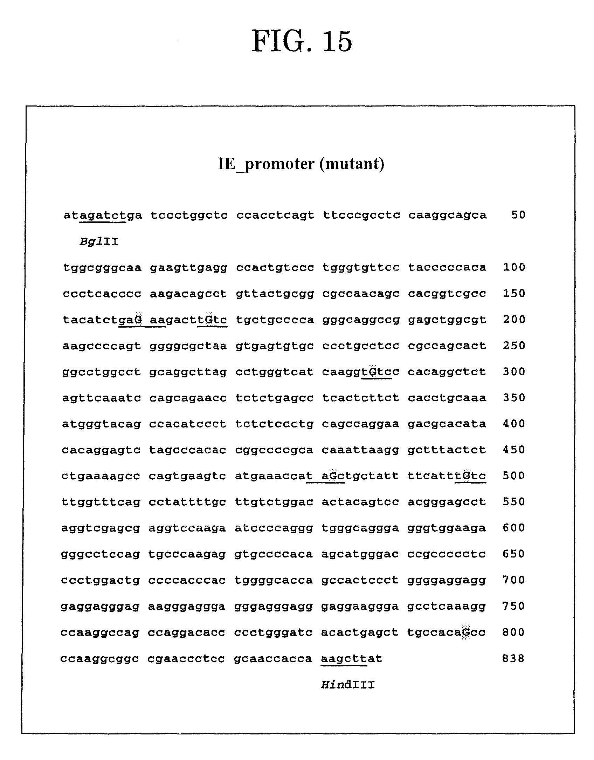

That is, an IE promoter (mutant) of about 0.84kb in which a mutation was introduced at the 5 sites of the GATA sequence in the IE promoter region of GATA-1 gene was artificially constructed by means of PCR. In this procedure, pGL3-HSI-IE DNA prepared in Example 4 was used as a template in the first PCR, and using synthetic primers corresponding to the both ends of the promoter region and the respective sequences having the mutation, fragments to be placed between the terminals of the region or between the respective sites having the mutation were amplified. Thus, the resulting PCR products were mixed and used as templates in the subsequent PCR reaction to select appropriate members as synthetic primers and amplify their longer region. By repeating this PCR, a PCR fragment corresponding to the total IE promoter region was obtained using the synthetic primers corresponding to both terminals of the promoter region. This fragment was inserted into pCR4-TOPO (Invitrogen) to determine the base sequence. Thus, the mutation was confirmed to be introduced as designed. Fig. 15 shows the sequence of the IE promoter (mutant) thus confirmed. The portion of the mutation is indicated by underlines, and both terminals have the *Bgl*II site and *Hind*III site as introduced. The BglII-HindIII fragment having the total IE promoter (mutant) was inserted between the BglII site and the HindIII site of pGL3 to give pGL3-IE promoter (mutant).

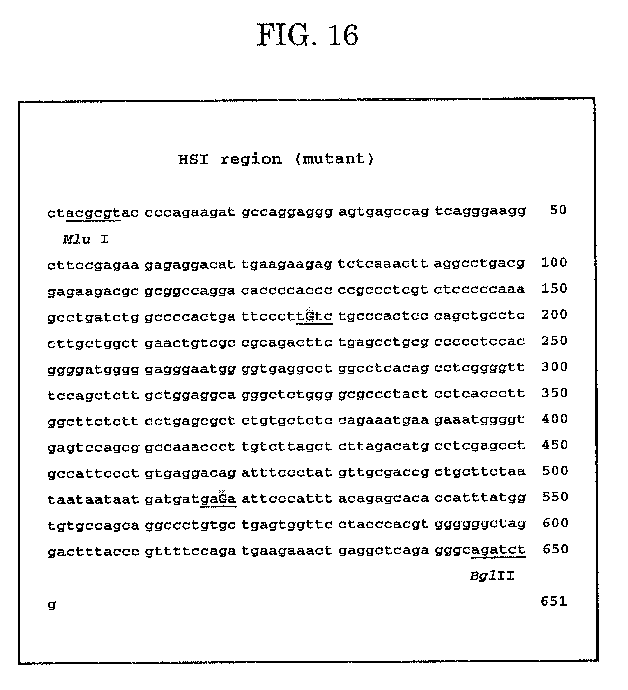

By the same PCR technique, the HSI (mutant) region of about 0.65kb in which a mutation was introduced at the 2 sites of the GATA sequence of the HSI region in GATA-1 gene was artificially constructed using pGL3-HSI-IE DNA prepared in Example 4 as a template in the first PCR. The resulting PCR fragment was inserted into pCR4-TOPO (Invitrogen) to determine the base sequence and confirm the mutation introduced. Fig. 16 shows the sequence of the HSI (mutant) region thus confirmed. The portion of the mutation is indicated by underlines, and both terminals respectively have the *Mlu*I site and *Bgl*II site as introduced. The MluI-BglII fragment having the total HSI (mutant) region was inserted between the MluI site and the BglII site of pTAL-Luc to give pTAL-HSI(mutant)-Luc. In addition, the KpnI-BglII fragment having the total HSI (mutant) region of pTAL-HSI(mutant)-Luc was inserted between KpnI-BglII of pGL3-IEpromoter (mutant) to give pGL3-HSI-IE Promoter (mutant) having the total IE promoter (mutant) and the total HSI (mutant) region.

### [Example 13] Effect of an HDAC inhibitor on the GATA-1 transcriptional activity

Using 15µg each of pGL3-HSI-IE Promoter prepared in Example 4 and pGL3-HSI-IE Promoter (mutant) prepared in Example 12, HEL cells (8.75x10⁶ cells) were transformed by electroporation (Voltage 1,750V, Charge 10µF, 365µL). After transformation, the transformants respectively were suspended in 2 mL of 10% FBS RPMI1640, and distributed to a 96-well white plate at a rate of 50µL/well. The plate was incubated at 37°C under 5% CO₂ in a condition of saturated humidity for 3 hours, and then 10%FBS RPMI1640 medium containing an HDAC inhibitor, i.e., Compounds A, C or D at a concentration of 2 times over the final concentration was added at a rate of 50µL/well. After incubation at 37°C under 5% CO₂ in a condition of saturated humidity for 8 hours, the luciferase activity in the cells was measured with a multi-label counter according to a manual of Bright-Glo™ Luciferase Assay System (Promega Corporation). As a result, it was found that luciferase was derived from pGL3-HSI-IE Promoter and pGL3-HSI-IE Promoter (mutant), respectively, depending on the GATA-1 promoter, but the activity derived from the mutant promoter was lower than that of the wild-type promoter. In addition, the luciferase activity derived from the mutant promoter was not inhibited by Compounds A, C and D differing from the wild-type (Table 5).

The above results indicate that the HDAC inhibitors, Compounds A, C and D, inhibit the function of transcriptional control system involved in the GATA sequence on the GATA-1 promoter to inhibit the function of transcriptional activation. The GATA sequence is known to be recognized by GATA-1 itself; this fact suggests that Compounds A, C and D might inhibit the function of the transcriptional activation of GATA-1 factor itself.

**Table 5**

| | | Relative luciferase activity (RLU) | |
|---|---|---|---|
| | | Wild type GATA-1 promoter | Mutant GATA-1 promoter |
| Compound A | (+) | 211 ± 13 | 301 ± 30 |
| | (-) | 586 ± 44 | 317 ± 7 |
| Compound C | (+) | 218 ± 5 | 311 ± 8 |
| | (-) | 609 ± 43 | 326 ± 11 |
| Compound D | (+) | 203 ± 17 | 290 ± 23 |
| | (-) | 681 ± 68 | 335 ± 10 |
| Mean ± Standard error (n=3) | | | |

### Industrial Applicability

According to the method of the invention, it is possible to rapidly screen a compound having a strong IL-2 transcription inhibitory activity simultaneously with a weak GATA-1 transcription inhibitory activity, by using reporter assay systems using 2 species of cells. It is also possible to rapidly screen a compound having a weak GATA-1 transcription inhibitory activity from HDAC inhibitors. The compounds selected by such a method are expected to be candidates for creating highly safe drugs with less thrombocytopenia effect in much higher probability than before. Thus, the method of the invention is very useful in studies for creation of new drugs.

## Claims

1. A method for selecting an immunosuppressive agent with a less thrombocytopenia effect, said method comprising the following items (1) and (2):
(1) selecting compounds having an immunosuppressive activity; and
(2) selecting a compound having a weak GATA-1 transcription inhibitory activity from the compounds selected in (1).

2. A method for selecting an immunosuppressive agent with a less thrombocytopenia effect, said method comprising the following items (1) to (3):
(1) measuring an immunosuppressive activity of an analyte;
(2) measuring a GATA-1 transcription inhibitory activity of the analyte; and
(3) comparing the immunosuppressive activity determined in (1) with the GATA-1 transcription inhibitory activity determined in (2) to select an immunosuppressive agent with a less thrombocytopenia effect.

3. A method for selecting an immunosuppressive agent with a less thrombocytopenia effect, said method comprising the following items (1) to (3):
(1) measuring an IL-2 transcription inhibitory activity in a test cell in the coexistence of the test cell and an analyte;
(2) measuring an GATA-1 transcription inhibitory activity in a test cell in the coexistence of the test cell and an analyte; and
(3) comparing the IL-2 transcription inhibitory activity determined in (1) with the GATA-1 transcription inhibitory activity determined in (2) to select an immunosuppressive agent with a less thrombocytopenia effect.

4. A method for selecting an immunosuppressive agent with a less thrombocytopenia effect, said method comprising the following items (1) to (3):
(1) measuring an IL-2 transcription inhibitory activity in a test cell into which an IL-2 reporter gene has been introduced in the coexistence of the test cell and an analyte;
(2) measuring a GATA-1 transcription inhibitory activity in a test cell into which a GATA-1 reporter gene has been introduced in the coexistence of the test cell and an analyte; and
(3) comparing the IL-2 transcription inhibitory activity determined in (1) with the GATA-1 transcription inhibitory activity determined in (2) to select an immunosuppressive agent with a less thrombocytopenia effect.

5. A method for selecting an immunosuppressive agent with a less thrombocytopenia effect as claimed in Claim 4, comprising measuring a (IL-2 IC50) value as an IL-2 transcription inhibitory activity, measuring a (GATA-1 IC50) value as a GATA-1 transcription inhibitory activity, and comparing both the values.

6. A method for selecting an immunosuppressive agent with a less thrombocytopenia effect as claimed in Claim 5, comprising selecting a compound having the (GATA-1 IC50) / (IL-2 IC50) value of 5 or more.

7. A method as claimed in any of Claims 4 to 6, wherein the GATA-1 reporter gene comprises the transcriptional control region of human GATA-1 gene and a reporter gene.

8. A method as claimed in any of Claims 4 to 7, wherein the GATA-1 reporter gene comprises a sequence of the region from -3769 to -3133 upstream of the transcription initiation point and sequence of the region from -789 to +30 proximal to the transcription initiation point of human GATA-1 gene.

9. A method as claimed in any of Claims 4 to 8, wherein the IL-2 reporter gene comprises the transcriptional control region of IL-2 gene and a reporter gene.

10. A method as claimed in any of Claims 4 to 9, wherein the IL-2 reporter gene comprises a sequence of the region from -378 to +54 proximal to the transcription initiation point of human IL-2 gene.

11. A method as claimed in any of Claims 4 to 10, wherein the test cell into which a GATA-1 reporter gene is introduced is a human megakaryocytic cell strain.

12. A method as claimed in any of Claims 4 to 11, wherein the test cell into which an IL-2 reporter gene is introduced is a human T cell-derived cell strain stimulated by phorbol 12-myristate 13-acetate, ionomycin and anti-CD28 antibody, and the test cell into which a GATA-1 reporter gene is introduced is a human megakaryocytic cell strain.

13. A method as claimed in Claim 12, wherein the human T cell-derived cell strain is a Jurkat cell.

14. A method as claimed in Claim 11, wherein the human megakaryocytic cell strain is a HEL cell.

15. A method as claimed in Claim 12, wherein the human megakaryocytic cell strain is a HEL cell.

16. A method as claimed in any of Claims 4 to 15, wherein the reporter gene is a firefly luciferase gene.

17. A method for selection as claimed in any of Claims 1 to 16, wherein the analyte is an HDAC inhibitor.

18. A method for selecting an immunosuppressive agent with a less thrombocytopenia effect, comprising measuring the amount of expression of GATA-1 protein.

19. A method for selecting an immunosuppressive agent with a less thrombocytopenia effect, said method comprising the following items (1) to (3):
(1) measuring the amount of expression of IL-2 protein;
(2) measuring the amount of expression of GATA-1 protein; and
(3) comparing both the amounts of expression to select an immunosuppressive agent with a less thrombocytopenia effect.

20. A kit for measurement in selecting an immunosuppressive agent with a less thrombocytopenia effect, comprising the following items (1) and (2):
(1) a DNA construct containing a GATA-1 reporter gene; and
(2) a test cell of megakaryocytic cell line.

21. A kit for measurement in selecting an immunosuppressive agent with a less thrombocytopenia effect, comprising the following items (1) to (4):
(1) a DNA construct containing an IL-2 reporter gene;
(2) a DNA construct containing a GATA-1 reporter gene;
(3) a test cell of T-cell line; and
(4) a test cell of megakaryocytic cell line.

22. An HDAC inhibitor with a less thrombocytopenia effect, said inhibitor being selected by a method as claimed in Claim 17.

23. An immunosuppressive agent with a less thrombocytopenia effect, said agent being selected by a method for selection as claimed in any of Claims 1 to 16.

24. An immunosuppressive agent for treatment of inflammatory disorders, diabetes mellitus, diabetic complications, homozygous thalassemia, fibrosis, cirrhosis, acute promyelocytic leukemia, protozoal infections, organ transplant rejection, autoimmune diseases, and tumors, said agent comprising as an active ingredient an HDAC inhibitor as claimed in Claim 22.

25. A therapeutic agent for treatment of inflammatory disorders, diabetes mellitus, diabetic complications, homozygous thalassemia, fibrosis, cirrhosis, acute promyelocytic leukemia, protozoal infections, organ transplant rejection, autoimmune diseases, and tumors, said agent comprising as an active ingredient an immunosuppressive agent as claimed in Claim 23.

26. A therapeutic method for treatment of inflammatory disorders, diabetes mellitus, diabetic complications, homozygous thalassemia, fibrosis, cirrhosis, acute promyelocytic leukemia, protozoal infections, organ transplant rejection, autoimmune diseases, and tumors, said method comprising administering an immunosuppressive agent with a less thrombocytopenia effect containing as an active ingredient an HDAC inhibitor as claimed in Claim 22.

27. A therapeutic method for treatment of inflammatory disorders, diabetes mellitus, diabetic complications, homozygous thalassemia, fibrosis, cirrhosis, acute promyelocytic leukemia, protozoal infections, organ transplant rejection, autoimmune diseases, and tumors, said method comprising administering an immunosuppressive agent with a less thrombocytopenia effect containing as an active ingredient an immunosuppressive agent as claimed in Claim 23.

28. Use of an HDAC inhibitor as claimed in Claim 22 in manufacture of a therapeutic agent for treatment of inflammatory disorders, diabetes mellitus, diabetic complications, homozygous thalassemia, fibrosis, cirrhosis, acute promyelocytic leukemia, protozoal infections, organ transplant rejection, autoimmune diseases, and tumors.

29. Use of an immunosuppressive agent as claimed in Claim 23 in manufacture of a therapeutic agent for treatment of inflammatory disorders, diabetes mellitus, diabetic complications, homozygous thalassemia, fibrosis, cirrhosis, acute promyelocytic leukemia, protozoal infections, organ transplant rejection, autoimmune diseases, and tumors.
